(19) Europäisches Patentamt
European Patent Office
Office européen des brevets

(11) Veröffentlichungsnummer: **0 234 196 A1**

(12) **EUROPÄISCHE PATENTANMELDUNG**

(21) Anmeldenummer: **87100174.9**

(22) Anmeldetag: **09.01.87**

(51) Int. Cl.⁴: **C07D 211/90**, C07D 413/04, C07D 409/04, C07D 401/04, C07D 405/04, C07D 417/04, A61K 31/445, //C07D295/18

(30) Priorität: **18.01.86 DE 3601397**

(43) Veröffentlichungstag der Anmeldung: **02.09.87 Patentblatt 87/36**

(84) Benannte Vertragsstaaten: **AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Franckowiak, Gerhard, Dr.**
**Henselweg 10**
**D-5600 Wuppertal 1(DE)**
Erfinder: **Thomas, Günther, Dr. c/o Bayer Italia S.p.A.**
**Rep. Pharmacologia Via delle Groane 126**
**I-20024 Garbagnate (Milano)(IT)**
Erfinder: **Schramm, Matthias, Dr.**
**Paffrather Strasse 38**
**D-5000 Köln 80(DE)**
Erfinder: **Kayser, Michael, Dr.**
**Fleyerstrasse 231**
**D-5800 Hagen(DE)**
Erfinder: **Gross, Rainer, Prof. Dr.**
**Platzhofstrasse 23**
**D-5600 Wuppertal 1(DE)**
Erfinder: **Bechem, Martin, Dr.**
**obere Bergerheide 4**
**D-5600 Wuppertal 1(DE)**

(54) **Substituierte 1,4-Dihydropyridin-3-carbonsäurepiperazide, Verfahren zu ihrer Herstellung und ihre Verwendung in Arzneimitteln.**

(57) 1,4-Dihydropyridin-3-carbonsäure-piperazide der allgemein Formel I

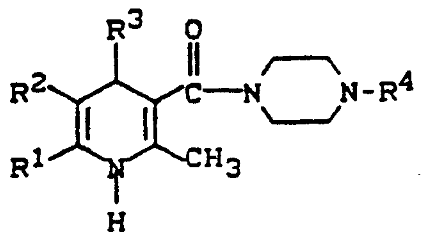

(I)

in welcher

EP 0 234 196 A1

$R^1$ -für Cyano
-für geradkettiges oder verzweigten $C_1$-$C_6$-Alkyl steht, das gegebenenfalls durch Halogen, Hydroxy oder $C_2$-$C_7$-Acyloxy substituiert ist,
$R^2$ -für Wasserstoff
-für Cyano,
-für Nitro oder
-für die Gruppe $CO_2R^5$ steht,
wobei
$R^5$ -geradkettiges oder verzweigtes, gegebenfalls substitutiertes $C_1$-$C_8$-Alkyl ist,
$R^3$ -für gegebenfalls substituiertes $C_6$-$C_{12}$-Aryl, oder
-für einen gegebenfalls substituiertes Heterocyclus aus der Reihe Pyridyl, Thienyl, Furyl, Pyrrolyl, Chinolyl, Isochinoly, Pyrimidyl, Benzoxadiazolyl, Imidazolyl, Thiazolyl, Oxazolyl, Chromenyl, oder Thiochromenyl steht, und

$R^4$ -für gegebenfalls substituiertes $C_6$-$C_{12}$-Aryl,
-für geradkettiges, verzweigtes oder cyclisches, gegebenfalls substituiertes $C_1$-$C_{20}$-Alkyl, $C_2$-$C_{20}$-Alkenyl oder $C_2$-$C_{20}$-Alkinyl
oder
-für die Gruppe $COR^6$ steht, wobei
$R^6$ -für Wasserstoff,
-für Hydroxy,
-für $C_1$-$C_8$-Alkoxy oder
-für die Gruppe

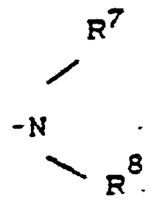

steht, wobei

$R^7$, $R^8$ -gleich oder verschieden sind und
-Wasserstoff,
-$C_1$-$C_8$-Alkyl,
-$C_6$-$C_{12}$-Aryl,
-$C_6$-$C_{14}$-Aralkyl oder
-$C_2$-$C_7$-Acyl bedeuten oder
$R^7$ und $R^8$ gemeinsam einen 5-7 gliedrigen gesättigen oder ungesättigen Ring bilden, der als weitere Heteratome Stickstoff, Schwefel und/oder Sauerstoff enthalten kann, mehrere Verfahren zur Herstellung sowie ihre Verwendung in Arzneimitteln.

# Substituierte 1,4-Dihydropyridin-3-carbonsäurepiperazide, Verfahren zu ihrer Herstellung und ihre Verwendung in Arzneimitteln

Die Erfindung betrifft 1,4-Dihydropyridin-3-carbonsäurepiperazide, Verfahren zu ihrer Herstellung sowie ihre Verwendung in Arzneimitteln.

Es ist bekannt, daß 1,4-Dihydropyridine gefäßerweiternde Eigenschaften besitzen und als Antihypertensiva verwendet werden können (vgl. DE-OS 2.629.892 und DE-OS 2.752.820).

Außerdem ist bekannt, daß Dihydropyridincarbonsäureamide eine Hemmung der Kontraktionskraft von glatten und kardialen Muskeln bewirken und zur Behandlung von Coronar-und Gefäßerkrankungen eingesetzt werden können (vgl. DE-OS 2.228.377).

Die Erfindung betrifft 1,4-Dihydropyridin-3-carbonsäurepiperazide der allgemeinen Formel I

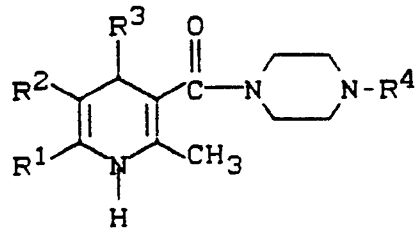

(I)

in welcher

$R^1$ -für Cyano oder

-für geradkettiges oder verzweigten $C_1$-$C_6$-Alkyl steht, das gegebenenfalls durch Halogen, Hydroxy oder $C_2$-$C_7$-Acyloxy substituiert ist,

$R^2$ -für Wasserstoff

-für Cyano,

-für Nitro oder

-für die Gruppe $CO_2R^5$ steht,

wobei

$R^5$ -geradkettiges oder verzweigtes $C_1$-$C_8$-Alkyl, bedeutet, das gegebenenfalls durch Hydroxy, Halogen, Cyano, $C_1$-$C_8$-Alkoxy, $C_1$-$C_8$-Alkylthio, Phenyl, Amino, $C_1$-$C_6$-Alkylamino, Di-$C_1$-$C_6$-alkylamino, Phenylamino, $C_2$-$C_7$-Acylamino oder $C_1$-$C_6$-Alkyl-benzyl-amino substituiert ist,

$R^3$ -für $C_6$-$C_{12}$-Aryl steht, das gegebenenfalls ein-oder mehrfach gleich oder verschieden durch Halogen, Nitro, Cyano, $C_1$-$C_8$-Alkyl, $C_1$-$C_8$Alkoxy, $C_1$-$C_8$-Alkylthio, $C_7$-$C_{14}$-Aralkyl, $C_7$-$C_{14}$-Aralkoxy oder $C_7$-$C_{14}$-Aralkyl thio, wobei die Arylreste jeweils wieder durch Nitro, Trifluormethyl, Cyano, Methoxy, Methylthio, Trifluormethoxy, Difluormethoxy, Halogen, oder $C_1$-$C_6$-Alkyl substituiert sein können, durch Trifluormethyl, Trifluormethylthio, Trifluormethoxy, Difluormethoxy, Amino, $C_1$-$C_6$-Alkylamino, Di-$C_1$-$C_6$-alkylamino oder durch $C_2$-$C_7$-Acylamino substituiert ist, oder

-für einen Heterocyclus aus der Reihe Pyridyl, Thienyl, Furyl, Pyrrolyl, Chinolyl, Isochinolyl, Pyrimidyl, Benzoxadiazolyl, Imidazolyl, Thiazolyl, Oxazolyl, Chromenyl, oder Thiochromenyl steht, wobei der Heterocyclus gegebenenfalls durch Phenyl, $C_1$-$C_6$-Alkyl, $C_1$-$C_6$-Alkoxy, $C_1$-$C_6$-Alkylthio, Trifluormethyl, Trifluormethoxy, Trifluormethylthio, Halogen, Amino, $C_1$-$C_6$-Alkylamino, Di-$C_1$-$C_6$ -alkylamino oder $C_2$-$C_7$-Acylamino substituiert ist,

und

$R^4$ -für $C_6$-$C_{12}$-Aryl steht, das gegebenenfalls ein-oder mehrfach gleich oder verschieden durch Halogen, Nitro, Cyano, $C_1$-$C_8$-Alkyl, $C_1$-$C_8$-Alkoxy, $C_1$-$C_8$-Alkylthio, Trifluormethyl, Trifluormethoxy, Trifluormethylthio, Difluormethoxy, Hydroxy, Amino, $C_1$-$C_6$-Alkylamino, Di-$C_1$-$C_6$-alkylamino oder $C_2$-$C_7$-Acylamino substituiert ist, oder

-für geradkettiges, verzweigtes oder cyclisches $C_1$-$C_{20}$-Alkyl, $C_2$-$C_{20}$-Alkenyl oder $C_2$-$C_{20}$-Alkinyl steht, das gegebenenfalls durch Halogen, $C_1$-$C_8$-Alkoxy, $C_1$-$C_8$-Alkylthio, Amino, $C_1$-$C_8$-Alkylamino, Di-$C_1$-$C_8$-alkylamino, $C_2$-$C_7$-Acylamino, Piperidino, Piperazino, Morpholino, Thiomorpholino, Pyrrolidino oder durch $C_6$-$C_{12}$-Aryl, wobei Aryl wiederum einen oder mehrere Substituenten aus der Gruppe Nitro, Cyano, Trifluormethyl, Halogen, $C_1$-$C_8$-Alkyl, $C_1$-$C_8$-Alkylthio oder $C_1$-$C_8$-Alkoxy tragen kann, oder durch die Gruppe $COR^6$ substituiert ist, wobei

$R^6$ -für Wasserstoff,

-für Hydroxy,

-für $C_1$-$C_8$-Alkoxy oder
-für die Gruppe

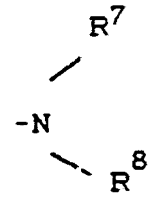

steht,
wobei
$R^7$, $R^8$ -gleich oder verschieden sind und
-Wasserstoff,
-$C_1$-$C_8$-Alkyl,
-$C_6$-$C_{12}$-Aryl
-$C_7$-$C_{14}$-Aralkyl oder
-$C_2$-$C_7$-Acyl bedeuten oder
$R^7$ und $R^8$ gemeinsam einen 5-7 gliedrigen gesättigten oder ungesättigten Ring bilden, der als weitere Heteroatome Stickstoff, Schwefel und/oder Sauerstoff enthalten kann,
oder
$R^4$ -für die Gruppe $COR^6$ steht, wobei $R^6$ die oben angegebene Bedeutung hat,
in Form ihrer Isomeren, Isomerengemische, Racemate, optischen Antipoden sowie deren physiologisch unbedenklichen Salze.

Bevorzugt sind Verbindungen der allgemeinen Formel I, in welcher
$R^1$ -für geradkettiges oder verzweigtes $C_1$-$C_4$-Alkyl steht, das gegebenenfalls durch ein oder mehrere Fluor, Chlor, Brom, Hydroxy, Benzoyloxy oder Acetyloxy substituiert ist,
$R^2$ -für Cyano,
-für Nitro oder
-für die Gruppe $CO_2R^5$ steht,
wobei
$R^5$ -geradkettiges oder verzweigtes $C_1$-$C_6$-Alkyl bedeutet, das gegebenenfalls durch ein oder mehrere Fluor, Chlor, Brom, Cyano, Phenyl, $C_1$-$C_6$-Alkoxy, $C_1$-$C_6$-Alkylthio, Amino, $C_1$-$C_4$-Alkylamino, Di-$C_1$-$C_4$-alkylamino, Acetylamino oder Benzylmethylamino substituiert ist,
$R^3$ -für Phenyl steht, das gegebenenfalls bis zu vierfach gleich oder verschieden durch Fluor, Chlor, Brom, Nitro, Cyano, $C_1$-$C_6$-Alkyl, $C_1$-$C_6$-Alkoxy, $C_1$-$C_6$-Alkylthio, Benzyl, Benzyloxy, Benzylthio, wobei die Phenylreste jeweils wiederum durch Nitro, Trifluormethyl, Trifluormethoxy, Trifluormethylthio, Methoxy, Fluor, Chlor, Brom, oder $C_1$-$C_4$-Alkyl substituiert sein können, oder durch Trifluormethyl, Trifluormethoxy, Difluormethoxy, Amino, $C_1$-$C_4$-Alkylamino, Di-$C_1$-$C_4$-alkylamino oder Acetylamino substituiert ist, oder
-für Pyridyl, Thienyl, Furyl, Pyrimidyl, Benzoxadiazolyl, 2-Phenyl-thiochromen-8-yl oder Chinolyl steht,
und
$R^4$ -für Phenyl steht, das gegebenenfalls bis zu vierfach gleich oder verschieden durch Fluor, Chlor, Brom, Nitro, Cyano, $C_1$-$C_6$-Alkyl, $C_1$-$C_6$-Alkoxy, $C_1$-$C_6$-Alkylthio, Trifluormethyl, Trifluormethoxy, Difluormethoxy, Hydroxy, Amino, $C_1$-$C_3$-Alkylamino, Di-$C_1$-$C_3$-alkylamino oder Acetylamino substituiert ist, oder
-für geradkettiges, verzweigtes oder cyclisches $C_1$-$C_{15}$-Alkyl, $C_2$-$C_{15}$-Alkenyl oder $C_2$-$C_{15}$-Alkinyl steht, das gegebenenfalls durch Fluor, Chlor, Brom, $C_1$-$C_6$-Alkoxy, $C_1$-$C_6$-Alkylthio, Amino, $C_1$-$C_6$-Alkylamino, Di-$C_1$-$C_6$-Alkylamino, Acetylamino, Benzoylamino oder durch Phenyl, wobei der Phenylrest wiederum ein bis drei Substituenten aus der Gruppe Nitro, Trifluormethyl, Fluor, Chlor, Brom, $C_1$-$C_6$-Alkyl, $C_1$-$C_6$-Alkoxy, $C_1$-$C_6$-Alkylthio tragen kann, oder durch die Gruppe $COR^6$ substituiert ist,
wobei
$R^6$ -für Wasserstoff,
-für Hydroxy,
-für $C_1$-$C_6$-Alkoxy oder
-für die Gruppe

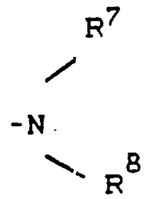

steht,
wobei
$R^7$, $R^8$ -gleich oder verschieden sind und
-für Wasserstoff,
-für $C_1$-$C_6$-Alkyl,
-für Phenyl,
-für Benzyl,
-für Acetyl oder für Benzoyl stehen
oder
$R^7$ und $R^8$ gemeinsam mit dem Stickstoffatom einen Heterocyclus aus der Reihe Pyrrolidin, Piperidin, Morpholin, Thiomorpholin, Imidazolidin, Piperazin oder Pyrrolin bilden,
oder
$R^4$ für die Gruppe der Formel $COR^6$ steht, wobei $R^6$ die oben angegebene Bedeutung hat,
in Form ihrer Isomeren, Isomerengemische, Racemate, opti schen Antipoden sowie deren physiologisch unbedenklichen Salze.

Besonders bevorzugte Verbindungen der Formel I sind solche, in welchen
$R^1$ für Methyl oder Ethyl steht, das gegebenenfalls durch Chlor, Brom oder Acetyloxy substituiert ist,
$R^2$ -für Cyano,
-für Nitro oder
-für die Gruppe $CO_2R^5$ steht,
wobei
$R^5$ geradkettiges oder verzweigtes $C_1$-$C_4$-Alkyl bedeutet, das gegebenenfalls durch ein oder mehrere Fluor, Cyano, $C_1$-$C_4$-Alkoxy, $C_1$-$C_4$-Alkylthio oder N-Benzylmethylamino substituiert ist,
$R^3$ -für Phenyl steht, das gegebenenfalls bis zu dreifach gleich oder verschieden durch Fluor, Chlor, Nitro, Cyano, $C_1$-$C_4$-Alkyl, $C_1$-$C_4$-Alkoxy, $C_1$-$C_4$-Alkylthio, Benzyl, Benzyloxy, Benzylthio substituiert ist, wobei die Phenylreste wiederum bis zu zweifach durch Nitro, Trifluormethyl, Methoxy, Fluor, Chlor, Methyl oder Ethyl substituiert sein können, oder durch Trifluormethyl oder Trifluormethoxy substituiert ist, oder
-für Pyridyl, Thienyl, Furyl, Pyrimidyl oder Benzoxadiazolyl oder 2-Phenyl-thiochromen-8-yl steht,
und
$R^4$ -für Phenyl steht, das gegebenenfalls bis zu zweifach gleich oder verschieden durch Fluor, Chlor, Nitro, $C_1$-$C_4$-Alkyl, $C_1$-$C_4$-Alkoxy, $C_1$-$C_4$-Alkylthio, Trifluormethoxy substituiert ist, oder
-für geradkettiges, verzweigtes oder cyclisches $C_1$-$C_{10}$-Alkyl, $C_2$-$C_{10}$-Alkenyl oder $C_2$-$C_{10}$-Alkinyl steht, das gegebenenfalls durch Fluor, Chlor, $C_1$-$C_4$-Alkoxy, $C_1$-$C_4$-Alkylthio, Amino, $C_1$-$C_4$-Alkylamino, Di-$C_1$-$C_4$-alkylamino, Acetylamino oder durch Phenyl, wobei Phenyl wiederum ein bis zwei Substituenten aus der Gruppe Nitro, Trifluormethyl, Fluor, Chlor, $C_1$-$C_4$-Alkoxy oder $C_1$-$C_4$-Alkyl tragen kann, oder durch die Gruppe $COR^6$ substituiert ist,
wobei
$R^6$ -für Wasserstoff,
-für Hydroxy,
-für $C_1$-$C_4$-Alkoxy oder
-für die Gruppe

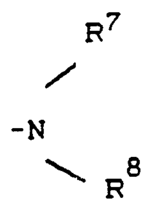

steht,
wobei
$R^7$, $R^8$ -gleich oder verschieden sind und

-für Wasserstoff,
-für $C_1$-$C_4$-Alkyl,
-für Phenyl,
-für Benzyl oder
-für Acetyl stehen, oder
$R^7$ und $R^8$ gemeinsam mit dem Stickstoffatom einen Heterocyclus aus der Reihe Pyrrolidin, Piperidin, Morpholin oder Thiomorpholin bilden, oder
$R^4$ -für die Gruppe $COR^6$ steht, wobei $R^6$ die oben angegebenen Bedeutung hat
in Form ihrer Isomeren, Isomerengemische, Racemate, optische Antipoden sowie deren physiologisch unbedenklichen Salze.

Physiologisch unbedenkliche Salze der erfindungsgemäßen Stoffe können Salze mit anorganischen oder organischen Säuren sein. Als Beispiele seien genannt: Hydrohalogenide, wie Hydrobromide, Hydrochloride, Hydrogensulfate, Sulfate, Hydrogenphosphate, Acetate, Maleate, Fumarate, Citrate, Tartrate, Lactate oder Benzoate.

Die erfindungsgemäßen Verbindungen der allgemeinen Formel I, in welcher
$R^1$ -$R^4$ die oben angegebene Bedeutung haben, erhält man, wenn man
[A] Aldehyde der allgemeinen Formel (II)

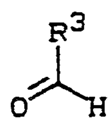

(II)

in welcher
$R^3$ die angegebene Bedeutung hat,
und Ketoverbindungen der allgemeinen Formel (III)

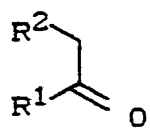

(III)

in welcher
$R^2$, $R^3$ die angegebene Bedeutung haben,
mit Ketoverbindungen der allgemeinen Formel (IV)

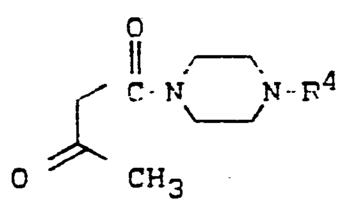

(IV)

in welcher
$R^4$ die angegebene Bedeutung hat,
und Ammoniak gegebenenfalls in Gegenwart von Wasser oder inerten organischen Lösungsmitteln umsetzt, oder wenn man
[B] Aldehyde der allgemeinen Formel (II) mit Ketoverbindungen der allgemeinen Formel (III) und Enaminen der allgemeinen Formel (V),

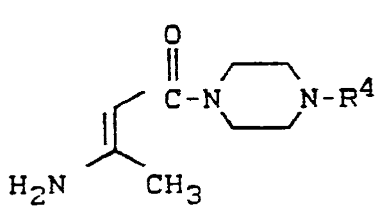

(V)

in welcher
$R^4$ die angegebene Bedeutung hat,
gegebenenfalls in Gegenwart von Wasser oder inerten organischen Lösungsmitteln umsetzt,
oder wenn man
[C] Aldehyde der allgemeinen Formel (II) mit Ketoverbindungen der allgemeinen Formel (IV) und Enaminen der allgemeinen Formel (VI),

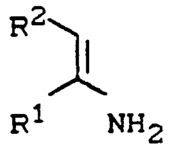

(VI)

in welcher
$R^1$, $R^2$ die angegebene Bedeutung haben,
gegebenenfalls in Gegenwart von Wasser oder inerten organischen Lösungsmitteln umsetzt,
oder wenn man
[D] Ketoverbindungen der allgemeinen Formel (III) und Ammoniak mit Ylidenverbindungen der allgemeinen Formel (VII)

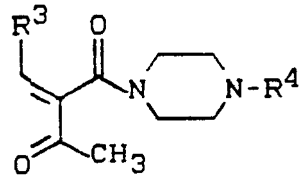

(VII)

in welcher
$R^3$, $R^4$ die oben angegebene Bedeutung haben, gegebenenfalls in Gegenwart von Wasser oder inerten organischen Lösungsmitteln umsetzt,
oder wenn man
[E] Ketoverbindungen der allgemeinen Formel (IV) und Ammoniak mit Ylidenverbindungen der allgemeinen Formel (VIII)

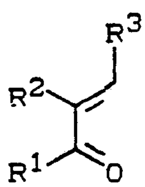

(VIII)

in welcher
$R^1$-$R^3$ die angegebene Bedeutung haben,
gegebenenfalls in Gegenwart von Wasser oder inerten organischen Lösungsmitteln umsetzt,
oder wenn man
[F] Enamine der allgemeinen Formel (VI) mit Ylidenverbindungen der allgemeinen Formel (VII) gegebenenfalls in Gegenwart von Wasser oder inerten organischen Lösungsmitteln umsetzt,
oder wenn man
[G] Enamine der allgemeinen Formel (V) mit Ylidenverbindungen der allgemeinen Formel (VIII) gegebenenfalls in Gegenwart von Wasser oder inerten organischen Lösungsmitteln umsetzt,
oder wenn man
[H] 1,4-Dihydropyridincarbonsäuren der allgemeinen Formel (IX),

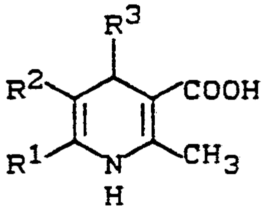

(IX),

in welcher
$R^1$-$R^3$ die angegebene Bedeutung haben,
nach bekannten Methoden, gegebenenfalls über ein reaktives Säurederivat, mit Verbindungen der allgemeinen Formel (X)

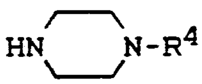

(X),

in welcher
$R^4$ die angegebene Bedeutung hat,
gegebenenfalls in Gegenwart eines inerten organischen Lösungsmittels umsetzt.

Als reaktive Säurederivate seien beispielhaft genannt : aktivierte Ester, Hydroxysuccinimidester, Säureimidazolide, Säurehalogenide, gemischte Anhydride, Umsetzung mit Cyclohexylcarbodiimid.

Je nach Art der verwendeten Ausgangsstoffe kann die Synthese der erfindungsgemäßen Verbindungen nach den Verfahren A-H durch folgende Formelschemata verdeutlicht werden:

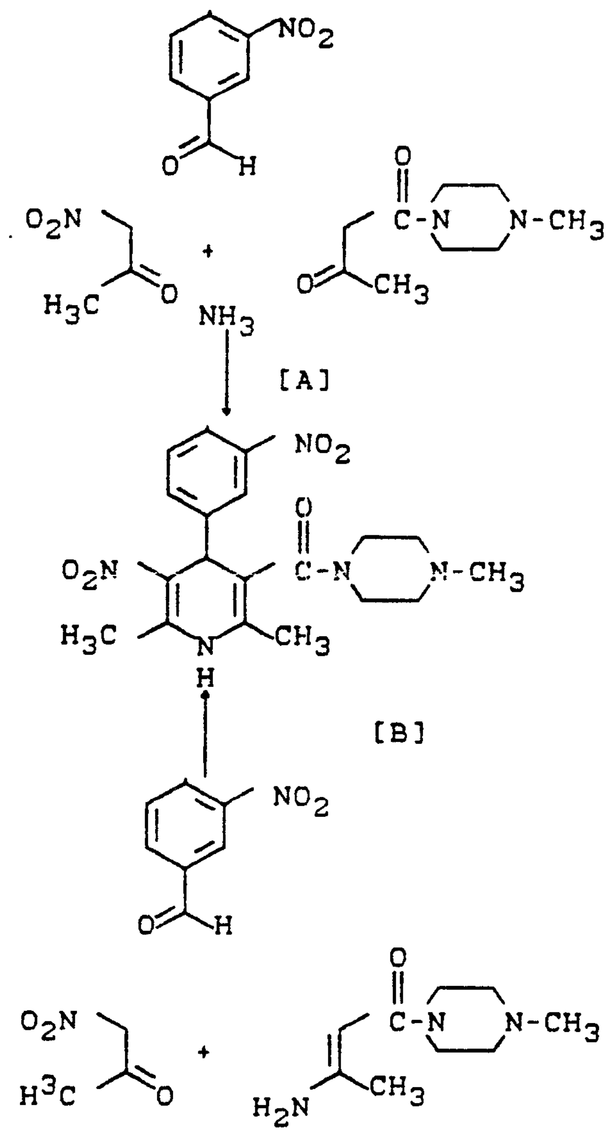
[A]
[B]

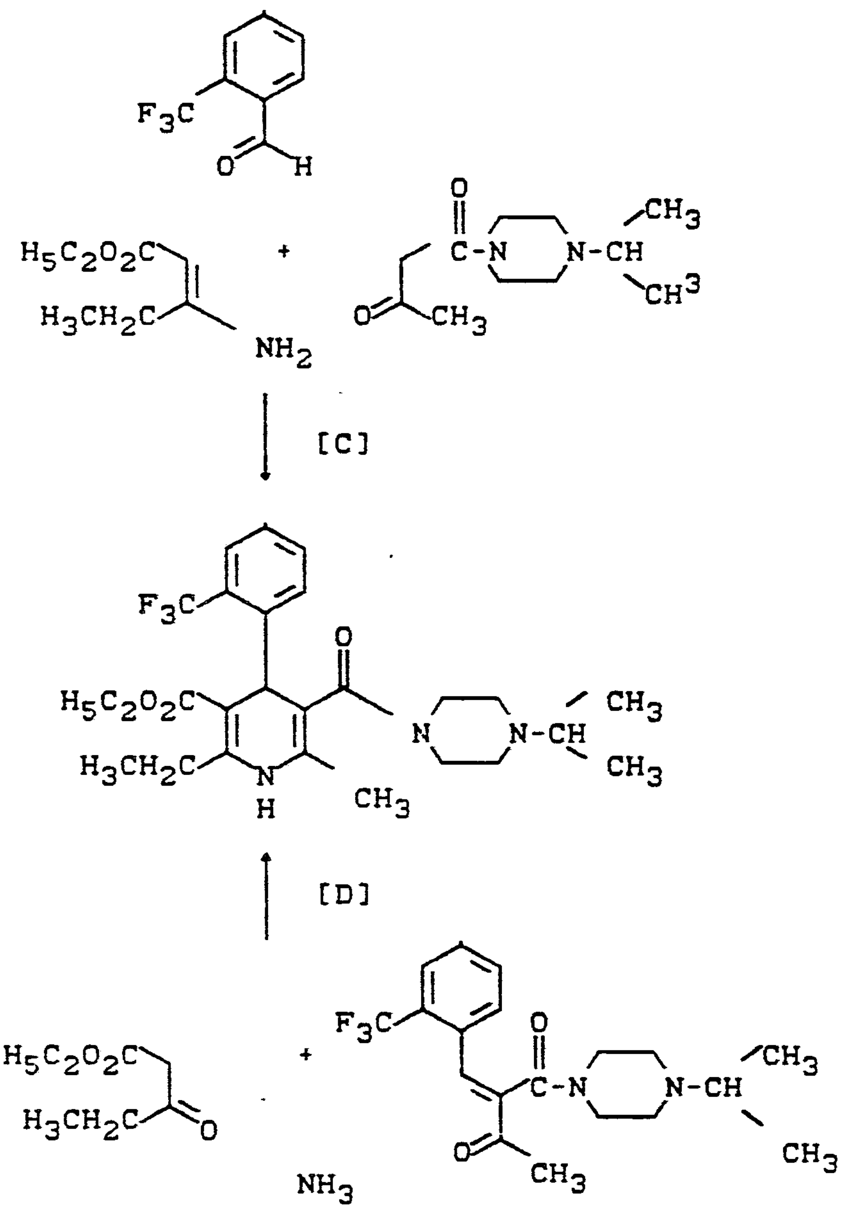
[C]
[D]

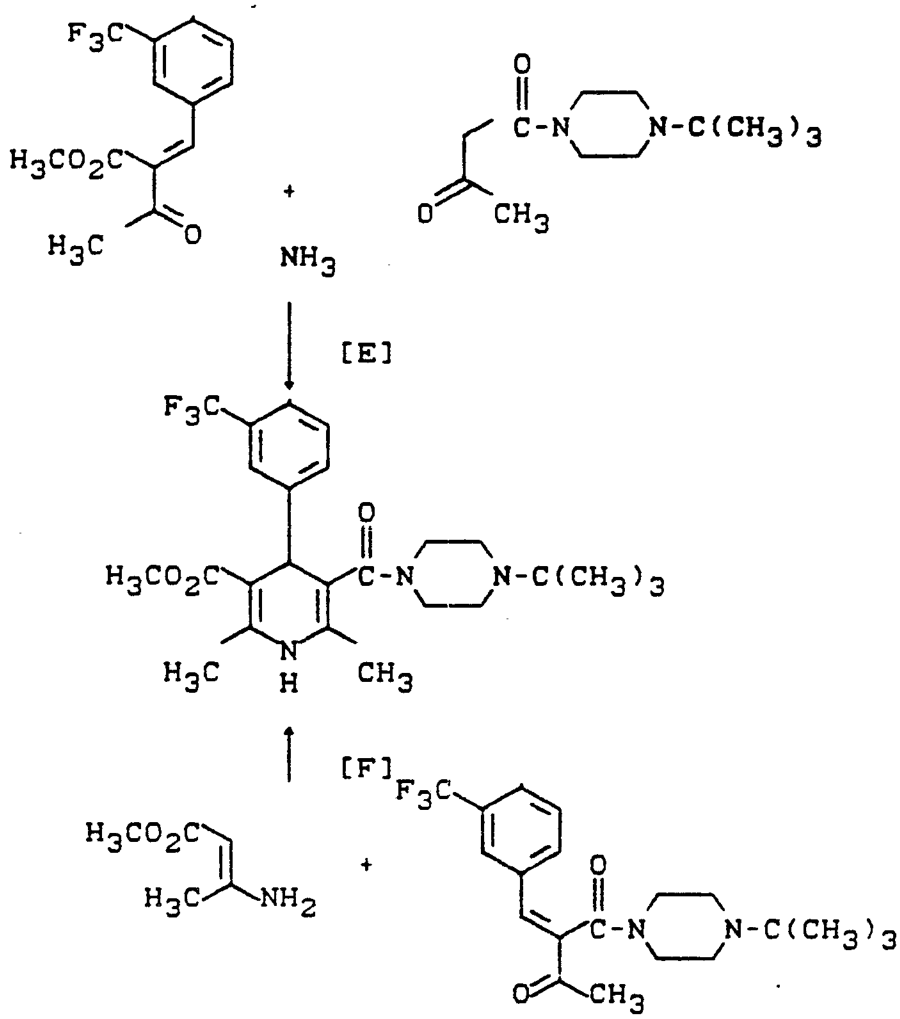
[E]
[F]

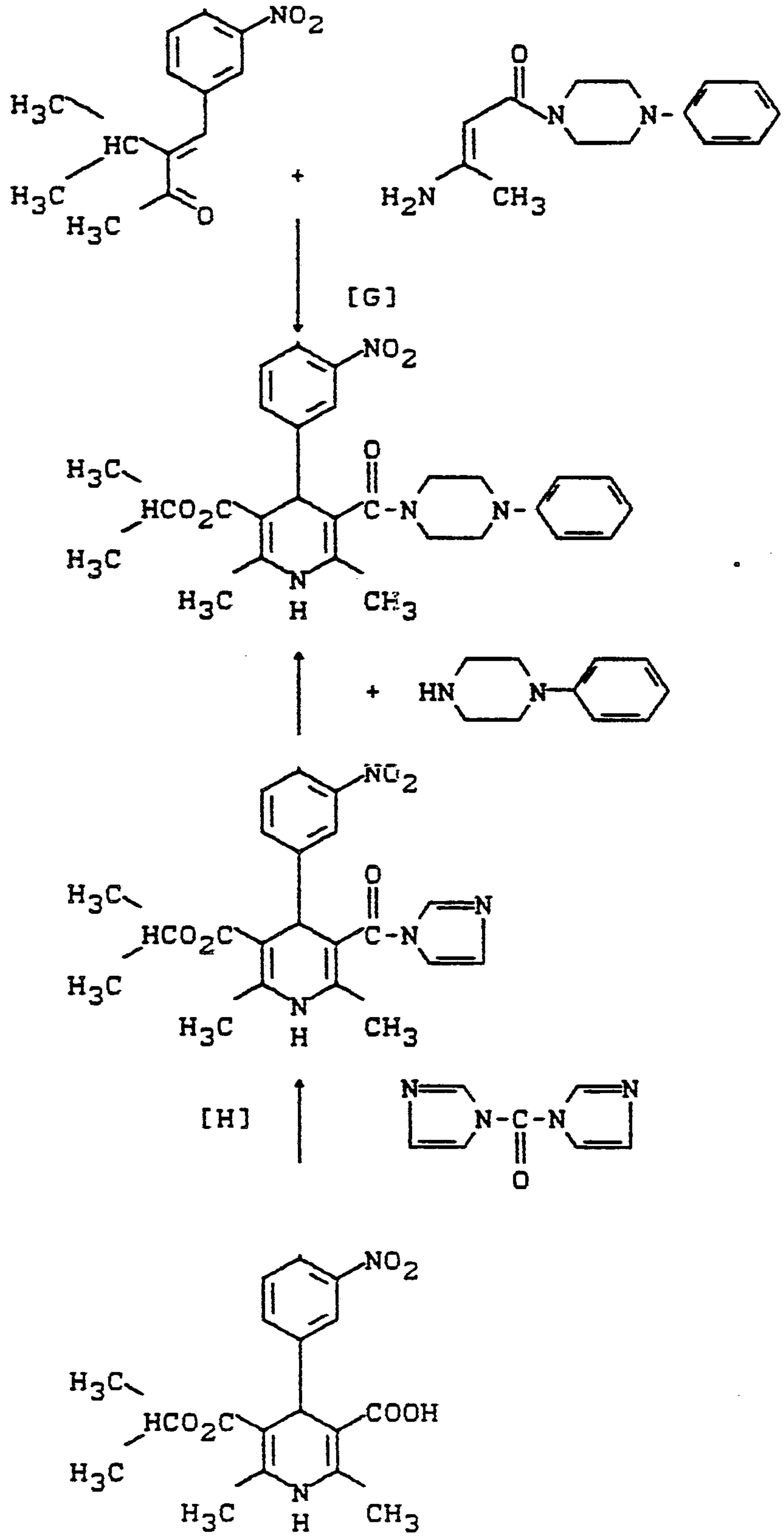


Die als Ausgangstoffe verwendeten Aldehyde der Formel (II) sind bekannt oder können nach bekannten Methoden hergestellt werden [vgl. E. Mosettig, Organic Reactions Vol.III, 218 ff. (1954); CA 59, 13929 - (1963)].

Die als Ausgangsstoffe verwendeten Ketoverbindungen der Formel (III) sind bekannt oder können nach bekannten Methoden hergestellt werden [vgl. D. Borrman, Houben-Weyls "Methoden der organischen Chemie" VII/4, 230 ff. (1968); Y. Oikawa, K. Sugano, O. Yonemitsu, J. Org. Chem. 43, 2087 (1978) ; N. Levy, C.W. Scaife, J. Chem Soc. (London) 1946, 1100; C.D. Hurd, M.E. Nilson, J. Org. Chem. 20, 927 - (1955), S. Gelin, P. Pollet, Synth. Commun. 1980, 805, Tetrahedron 34 1453 (1978)].

Die als Ausgangsstoffe verwendeten Enamine (VI) sind bekannt oder können nach literaturbekannten Methoden hergestellt werden [vgl. S.A. Glickmann, A.C. Cope, J. Chem Soc. 67, 1017 (1945), H. Böhme, K.-H. Weisel, Arch. Pharm. 310, 30 (1977)].

Die als Ausgangsstoffe verwendeten Ylidenverbindungen (VIII) sind bekannt oder können nach literaturbekannten Methoden hergestellt werden (vgl. G. Jones "The Knoevenagel Condensation", Organic Reactions XV, 204 ff. (1967); für $R^5$ = $NO_2$ vgl. W. Sassenberg A. Dornow, Liebigs Ann. Chem. 602, 14 (1957)].

Die als Ausgangsstoffe verwendeten 1.4-Dihydropyridincarbonsäuren (IX) sind bekannt oder können nach bekannten Methoden hergestellt werden (vgl. DE 28 47 237, DE 31 30 041, DE 33 19 956, BE 89 39 984, DE 33 31 808).

Die als Ausgangsstoffe verwendeten Piperazine (X) sind ebenfalls bekannt.

Die Verbindungen (IV), (V) und (VII) sind neu. Sie können nach literaturbekannten Methoden, wie in den Beispielen beschrieben, hergestellt werden. Beispielsweise ebenfalls nach:

D. Borrmann in Houben-Weyl, Methoden der organischen Chemie, Band VII/4, 230 ff. (1968); G. Jones in Organic Reactions Vol. VI, 204 ff (1967); S.A. Glickmann, A.C. Cope in J. Amer. Chem. Soc. 67, 1017 - (1945).

Als Verdünnungsmittel kommen für die Verfahren A-G Wasser oder alle inerten organischen Lösungsmittel in Frage. Hierzu gehören bevorzugt Alkohole wie Methanol, Ethanol n-bzw. iso-Propanol, Ether wie Diethylether, Tetrahydrofuran, Dioxan' oder Glykolmono-oder dimethylether, Eisessig, Pyridin, Dimethylformamid, Dimethylsulfoxid, Acetonitril, Hexamethylphosphorsäuretriamid oder Essigester. Ebenso können Gemische der genannten Lösungsmittel verwendet werden.

Für Verfahren H kommen die üblichen inerten organischen Lösungsmittel in Frage. Hierzu gehören bevorzugt chlorierte Kohlenwasserstoffe wie Dichlormethan, Trichlormethan, Tetrachlormethan oder 1,2-Dichlorethan oder Ether wie Diethylether, Tetrahydrofuran, Dioxan oder 1.2-Dimethoxyethan oder aromatische Kohlenwasserstoffe wie Benzol, Toluol, oder Xylol oder Acetonitril, Nitromethan, Dimethylformamid, Hexamethylphosphorsäuretriamid, Pyridin oder Essigsäureethylester.

Die Reaktionstemperaturen für alle Verfahren können in einem größeren Bereich variiert werden. Im allgemeinen arbeitet man bei Verfahren A bis G in einem Bereich von 10°C bis 200°C, bevorzugt von 20°C bis 150°C. Bei Verfahren H arbeitet man im allgemeinen in einem Bereich von -70°C bis +60°C, bevorzugt von -50°C bis +40°C.

Die Umsetzung kann bei Normaldruck, aber auch bei erhöhtem Druck durchgeführt werden. Im allgemeinen arbeitet man bei Normaldruck.

Bei der Durchführung der erfindungsgemäßen Verfahren ist das Verhältnis der an der Reaktion beteiligten Stoffe beliebig. Im allgemeinen arbeitet man jedoch mit molaren Mengen der Reaktanden. Bei Verfahren H hat es sich als zweckmäßig erwiesen, das Amin im bis zu 5fach molaren Überschuß einzusetzen.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die Substanzen beeinflussen den Kalziumhaushalt von eukariontischen Zellen. Sie sind damit geeignet als Koronartherapeutika und können herzprotektiv oder mykardstimulierend wirken. Darüber hinaus können sie den Blutdruck günstig beeinflussen und den Blutzucker normalisieren. Sie können einen Einfluß auf die Blutplättchenaggregation haben.

Die Koronar-und Herzwirkungen wurden am isolierten perfundierten Herzen von 200 g schweren Albino-Meerschweinchen beiderlei Geschlechts gefunden, das mit geeigneten Verdünnungen der Substanzen perfundiert wurde. Dazu wurden die Tiere getötet, der Thorax eröffnet, in die freipräparierte Aorta eine Metallkanüle eingebunden und der linke Vorhof eröffnet.

Das Herz wurde mit den Lungen aus dem Thorax herausgetrennt und über die Aortenkanüle an die Perfusionsapparatur bei laufender Perfusion angeschlossen, Die Lungen wurden an den Lungenwurzeln abgetrennt. Als Perfusionsmedium diente eine Krebs-Henseleit-Lösung (118 mMol/l NaCl, 4,8 mMol/l KCl, 1,2 mMol/l $MgSO_4$, 119 mMol/l $MgSO_4$, 25 mMol/l $NaHCO_3$, 0,013 mMol/l NaEDTA, pH 7,4, 10 mMol/l

Glukose) mit 1,2 mMol $CaCl_2$, die vor der Perfusion partikelfrei filtriert wurde. Die Herzen wurden mit konstantem Fluß mit 10 ml/min bei 32°C perfundiert. Die Herzkontraktionen wurde isovolumetrisch mit Hilfe eines in den linken Ventrikel eingeführten Latexballons gemessen und auf einem Schnellschreiber registriert. Als Maß für den Koronarwiderstand wurde der Perfusionsdruck registriert.

Die Wirkungen auf Kontraktilität und Koronarwiderstand einzelner Beispiele der erfindungsgemäßen Verbindungen sind in Tabelle 1 zusammengefaßt.

Tabelle 1:

| Bsp.-Nr. | Konz. (µg/ml) | prozentuale Veränderung von Kontrakt. | Koron.-Widerstand |
|---|---|---|---|
| 22 | 1 | -5 | -6 |
| | 10 | -52 | -19 |
| 25 | 1 | 0 | -10 |
| | 10 | -9 | -18 |
| 57 | 1 | -49 | -35 |
| | 10 | -93 | -38 |
| 54 | 1 | -61 | -66 |
| | 10 | -77 | -74 |
| 60 | 1 | -30 | -9 |
| | 10 | -76 | -12 |
| 17 | 1 | +28 | -40 |
| | 10 | +48 | -40 |

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermittel und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle - (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch-und Traubenzucker), Emulgiermittel (z.B. Polyoxyethylen-Fettsäure-Ester), Polyoxyethylen-Fettalkohol-Ether (z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendung gedacht sind, können die Wirkstoffe außer den obengenannten Hilfsstoffen mit verschiedenen Geschmackaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Herstellungsbeispiele

Beispiel 1

3-Aminocrotonsäure-(4-cyclohexylpiperazid)

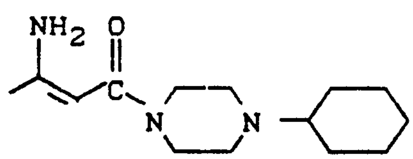

a) 3-Oxo-buttersäure-(4-cyclohexylpiperazid)

24 g (0.1 Mol) 1-Cyclohexylpiperazin werden in 150 ml absol. Tetrahydrofuran suspendiert. Man gießt 20 ml Triethylamin zu, tropft nach 20 min 18.6 g Diketen in 20 ml Tetrahydrofuran ein und rührt 2 h bei Raumtemperatur nach. Danach destilliert man einen Teil des Lösungsmittels im Vakuum ab, gießt den Ansatz auf 200 ml Eiswasser, gibt 20 g $Na_2CO_3$ zu und extrahiert 3 mal mit je 50 ml Methylenchlorid. Nach Trocknen der organischen Phase mit $MgSO_4$ erhält man als Eindampfrückstand rohes 3-Oxo-buttersäure-(4-cyclohexylpiperazid).

b) 3-Aminocrotonsäure-(4-cyclohexylpiperazid)

0.1 Mol rohes 3-Oxo-buttersäure-(4-cyclohexylpiperazid) wird in 150 ml Tetrahydrofuran gelöst und unter Rückfluß wird nach Zugabe von 0.5 g p-Toluolsulfonsäure 4 h Ammoniak eingeleitet Man läßt über Nacht bei Raumtemperatur stehen, destilliert das Lösungsmittel i.Vak. ab und kristallisiert den Rückstand aus Toluol/Petrolether.
Ausbeute: 18.6 g (74% der Theorie)
Schmelzpunkt: 92°C

Analog Beispiel 1 wurden die in Tabelle 1 aufgeführten Verbindungen synthetisiert.

Tabelle 1

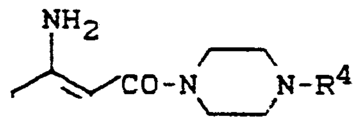

| Bsp.-Nr. | $R^4$ | Schmelzpunkt [°C] |
|---|---|---|
| 2 | 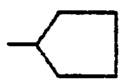 | 141° C |
| 3 | $-CH_2-CH(CH_3)_2$ | Oel |
| 4 | $-CH_2-CH=CH_2$ | Oel |
| 5 | $-CH_2-C\equiv CH$ | Oel |
| 6 | $CH(CH_3)_2$ | 208° C |
| 7 | 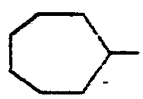 | 186° C |

Tabelle 1 (Fortsetzung)

| | | |
|---|---|---|
| 8 | 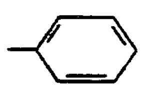 | 150° C |
| 9 | 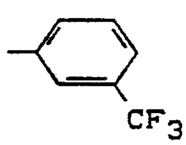 | 78° C |
| 10 | $-CH_2-CH_2$ 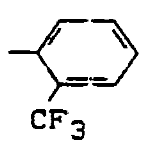 | Oel |
| 11 | $-CH_2-CO-$ 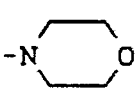 | 75° C |

Beispiel 12

1.4-Dihydro-2.6-dimethyl-5-nitro-4-(2-trifluormethylphenyl)-pyridin-3-carbonsäure-(4-cyclohexylpiperazid)

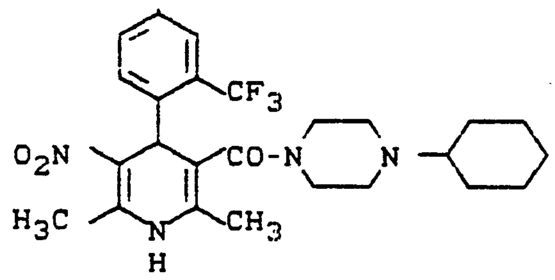

17.4 g (0.10 Mol) 2-Trifluormethylphenylbenzaldehyd, 12.0 g (0.12 Mol) Nitroaceton und 25.1 g (0.10 Mol) β-Aminocrotonsäure-(4-cyclohexylpiperazid) werden in 50 ml Isopropanol 12 h auf 60°C erhitzt. Beim Abkühlen fällt das Produkt aus und wird aus Isopropanol umkristallisiert.
Ausbeute: 23.6 g (48 % der Theorie)
Schmp.: 240°C (Zers.)

Beispiel 13

5-Carboxyethyl-1,4-dihydro-2,6-dimethyl-4-(3-nitrophenyl) pyridin-3-carbonsäure-(4-cyclopentylpiperazid)

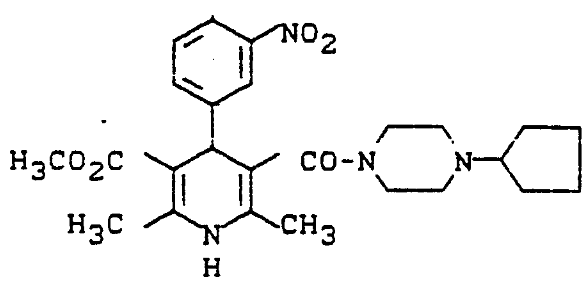

15.1 g (0.10 Mol) 3-Nitrobenzaldehyd, 25.2 g (0.10 Mol) Acetessigsäure-(4-cyclopentylpiperazid) und 12.9 g (0.10 Mol) β-Aminocrotonsäureisopropylester werden in 60 ml Isopropanol 12 h zum Rückfluß erhitzt. Das Produkt kristallisiert beim Anreiben. Rekristallisation aus Isopropanol.
Ausbeute: 29.4 g (61% der Theorie)
Schmp.: 130°C

Beispiel 14

5-Carboxymethyl-4-(2-chlorphenyl)-dihydro-2.6-dimethylpyridin-3-carbonsäure-(4-isopropylpiperazid)

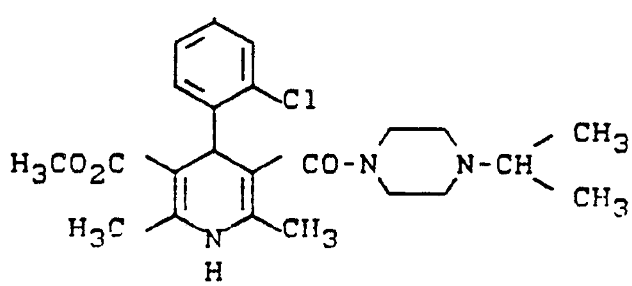

11.9 g (50 mMol) 2-Chlorbenzylidenacetessigsäuremethylester und 10.5 g (50 mMol) 3-Aminocrotonsäure-(4-isopropylpiperazid) werden in 50 ml Isopropanol 12 h zum Rückfluß erhitzt. Das Produkt kristallisiert nach dem Abkühlen.
Ausbeute: 1.7 g (79% der Theorie)
Schmp.: 214°C

Beispiel 15

4-(2,1,3-Benzoxadiazol-4-yl)-1,4-dihydro-2,6-dimethyl-5-nitro-pyridin-3-carbonsäure-[4-(3-trifluormethylphenyl)piperazid]

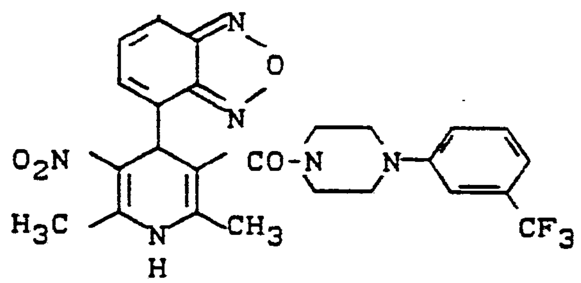

7.4 g (50 mMol) 2.1.3-Benzoxadiazol-4-aldehyd, 10 g (0.1 Mol) Nitroaceton und 15.6 g (50 mMol) 3-Aminocrotonsäure-[4-(3-trifluormethylphenyl)piperazid] werden in Isopropanol 6 h auf 60°C erhitzt. Das Produkt kristallisiert nach teilweisen Abdampfen des Lösungsmittels und wird aus wenig Isopropanol umkristallisiert.
Ausbeute: 8.9 g (34% der Theorie)
Schmp.: 219°C

Beispiel 16

4-(2-Benzyloxyphenyl)-1,4-dihydro-2,6-dimethyl-5-nitropyridin-3-carbonsäure-[4-(2-trifluormethylphenylethyl)piperazid]

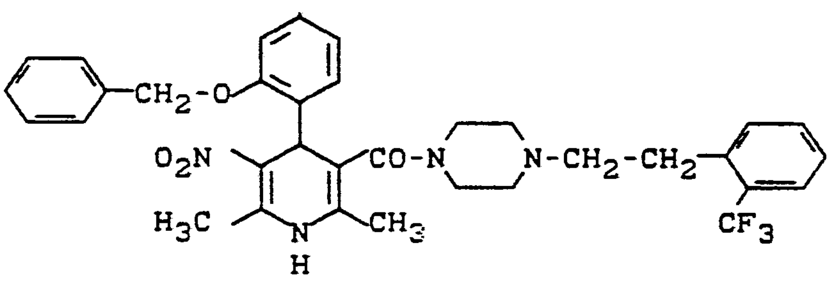

2,97 g (10 mMol) 1-(-2-Benzyloxyphenyl)-2-nitro-buten-1-on-3 und 2.96 g (mMol) 3-Aminocrotonsäure--[4-(2-trifluormethylphenylethyl)piperazid] werden in 15 ml Isopropanol 4 h auf 60°C erhitzt. Nach dem Erkalten erhält man ein Rohprodukt das aus wenig Ethanol umkristallisiert wird.
Ausbeute: 3.45 g (55% der Theorie)
Schmp.: 198°C

Beispiel 17

1,4-Dihydro-2,6-dimethyl-5-nitro-4-(3-nitrophenyl)pyridin-3-carbonsäure-(4-phenylpiperazid)

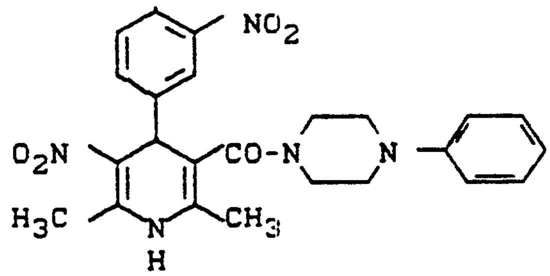

a) 2-Acetyl-3-(3-nitrophenyl)-2-propensäure-(4-phenylpiperazid

24.6 g (0.1 Mol) 3-Oxo-buttersäure-(4-phenylpiperazid) werden in 60 ml Acetanhydrid mit 20.6 g (0.1 Mol) 1-n-Butyliminomethyl-3-nitrobenzol zur Reaktion gebracht. Der Ansatz wird anschließend auf Eiswasser gegossen und hydrolysiert. Das ausfallende Öl wird in Methylenchlorid aufgenommen, mit verdünnter Bicarbonatlösung und Wasser gewaschen, mit Magnesiumsulfat getrocknet. Nach Abdampfen des Lösungsmittels erhält man eine rohe Benzylidenverbindung die so eingesetzt wird.

b) 1.4-Dihydro-2.6-dimethyl-5-nitro-4-(3-nitrophenyl)pyridin-3-carbonsäure-(4-phenylpiperazid)

2.5 g (10 mMol) rohes 2-Acetyl-3-(3-nitrophenyl)-2-propensäure-(4-phenyl-piperazid) und 2.5 g (25 mMol) 2-Amino-1-nitro-1-propen werden in 15 ml Isopropanol 6 h auf 60°C erhitzt. Das ausgefallene Rohprodukt wird aus Isopropanol kristallisiert.
Ausbeute: 2.7 g (58 % der Theorie)
Schmp.: 200°C

Beispiel 18

5-Carboxymethyl-4-(2-chlorphenyl)-1,4-dihydro-2,6-dimethylpyridin-3-carbonsäure-(4-phenyl-piperazid)

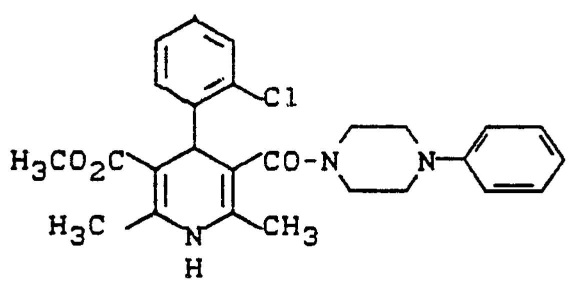

Zu 1.60 g (5 mMol) 5-Carboxymethyl-4-(2-chlorphenyl)-1,4-dihydro-2,6-dimethyl-pyridin-3-carbonsäure in 20 ml absol. Tetrahydrofuran suspendiert werden 1.1 g (6.5 mMol) Carbonyldiimidazol gegeben. Es wird 30 min bei Raumtemperatur und 30 min bei Rückflußtemperatur gerührt. Zu der erhaltenen Lösung werden 1.62 g (10 mMol) Phenylpiperazin in 5 ml absol. Tetrahydrofuran gegeben. Diese Mischung wird 3.5 h zum Ruckfluß erhitzt, anschließend eingeengt, im Methylenchlorid aufgenommen, nacheinander mit 1 n Salzsäure, 1 n Natronlauge und Wasser gewaschen, mit Magnesiumsulfat getrocknet und wieder eingeengt. Der Rückstand kristallisiert aus Isopropanol.
Ausbeute: 1.82 g (78 % der Theorie)
Schmp.: 182°C

Die analog den vorstehenden Beispielen 12 bis 18 hergestellten 1.4-Dihydropyridin-3-carbonsäurepiperazide sind in den Tabellen 2 und 3 zusammengefaßt.

Tabelle 2

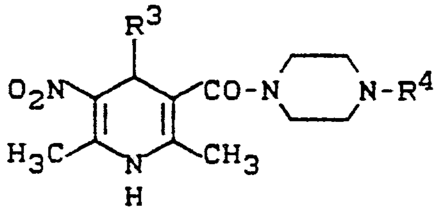

| Bsp.-Nr. | $R^3$ | $R^4$ | Schmp. (° C) |
|---|---|---|---|
| 19 | 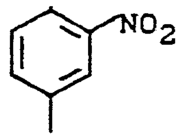 | 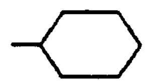 | 228 (Z) |
| 20 | 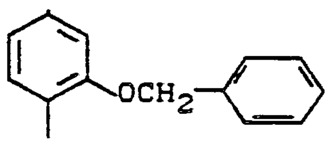 | 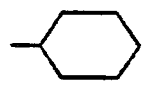 | 117 (Z) |
| 21 | 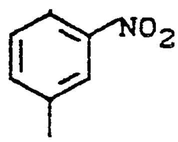 | 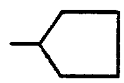 | 228 (Z) |
| 22 | 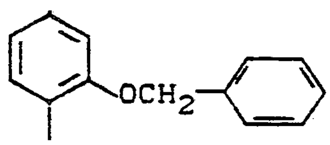 | 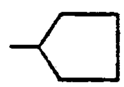 | 203 (Z) |
| 23 | 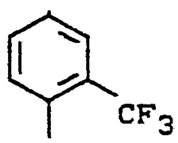 | 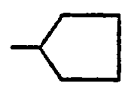 | 261 (Z) |
| 24 | 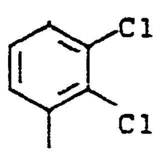 | 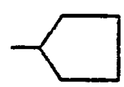 | 278 (Z) |
| 25 | 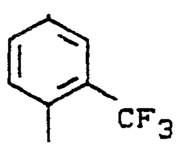 | $-CH_2-CH(CH_3)_2$ | 248 |

Tabelle 2 (Fortsetzung)

| Bsp. Nr. | $R^3$ | $R^4$ | Schmp. (°C) |
|---|---|---|---|
| 26 | 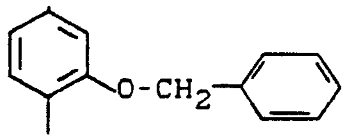 | $-CH_2-CH(CH_3)_2$ | 218 |
| 27 | 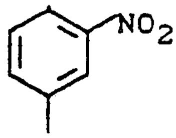 | $-CH_2-CH(CH_3)_2$ | 225 (Z) |
| 28 | 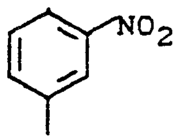 | $-CH_2-CH=CH_2$ | 190 |
| 29 | 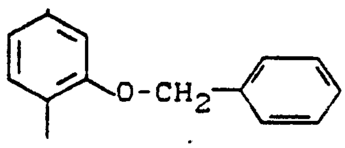 | $-CH_2-CH=CH_2$ | 223 (Z) |
| 30 | 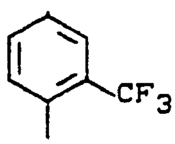 | $-CH_2-CH=CH_2$ | 263 (Z) |
| 31 | 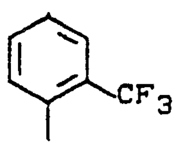 | $-CH_2-C\equiv CH$ | 233 (Z) |
| 32 | 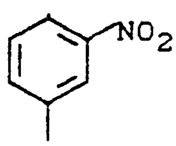 | $-CH_2-C\equiv CH$ | 176 |
| 33 | 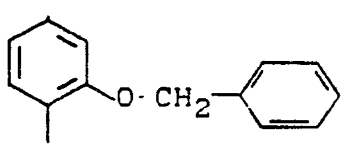 | $-CH_2-C\equiv CH$ | 214 |

Tabelle 2 (Fortsetzung)

| Bsp. Nr. | $R^3$ | $R^4$ | Schmp. (°C) |
|---|---|---|---|
| 34 | 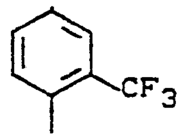 | $-CH_2(CH_3)_2$ | 262 (Z) |
| 35 | 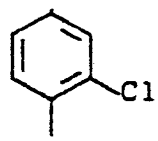 | $-CH(CH_3)_2$ | 250 (Z) |
| 36 | 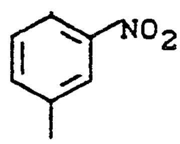 | $-CH(CH_3)_2$ | 200 (Z) |
| 37 | 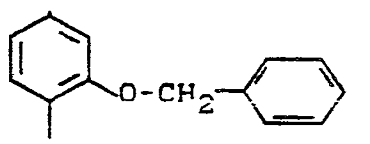 | $-CH(CH_3)_2$ | 227 |
| 38 | 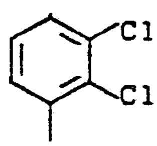 | $-CH(CH_3)_2$ | 271 (Z) |
| 39 | 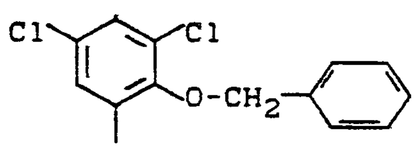 | $-CH(CH_3)_2$ | Harz |
| 40 | 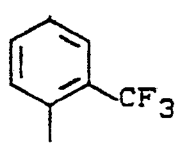 | 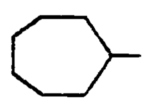 | 274 (Z) |
| 41 | 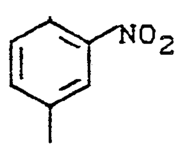 | 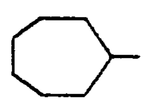 | 187 |

Tabelle 2 (Fortsetzung)

| Bsp. Nr. | $R^3$ | $R^4$ | Schmp. (°C) |
|---|---|---|---|
| 42 | 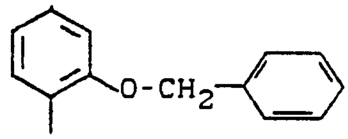 | 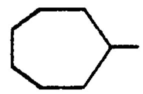 | 212 |
| 43 | 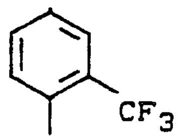 | 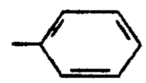 | 273 |
| 44 | 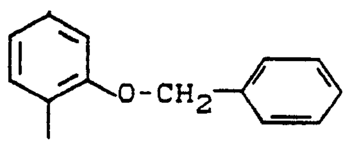 | 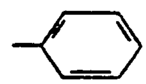 | 249 |
| 45 | 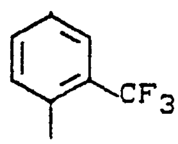 | 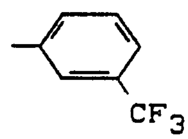 | 265 (Z) |
| 46 | 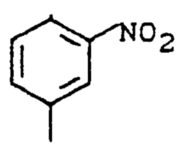 | 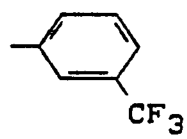 | 121 |
| 47 | 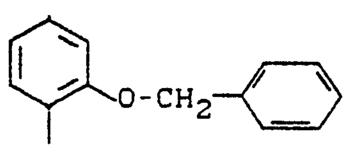 | 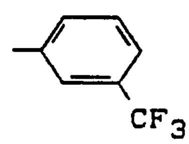 | 183 |
| 48 | 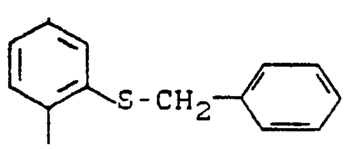 | 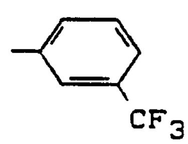 | 195 (Z) |

<u>Tabelle 2</u> (Fortsetzung)

| Bsp. Nr. | $R^3$ | $R^4$ | Schmp. (° C) |
|---|---|---|---|
| 49 | 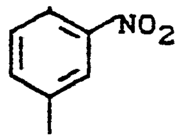 | $-CH_2-CH_2-$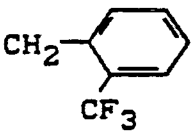 | 118 |
| 50 | 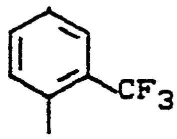 | $-CH_2-CH_2-$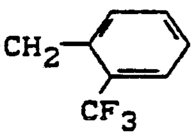 | 266 (Z) |
| 51 | 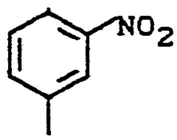 | $-CH_2-CO-$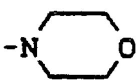 | 165 |
| 52 | 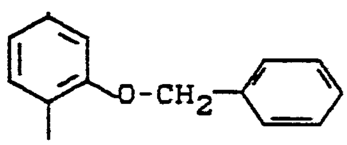 | $-CH_2-CO-$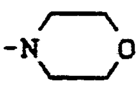 | 173 |
| 53 | 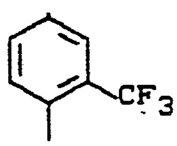 | $-CH_2-CO-$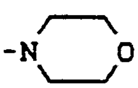 | 163 |

Tabelle 3

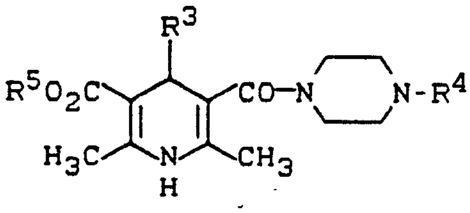

| Bsp. Nr. | $R^3$ | $R^4$ | $R^5$ | Schmp. (°C) |
|---|---|---|---|---|
| 54 | 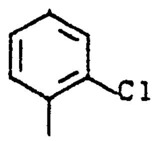 | 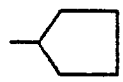 | $CH_3$ | 186 |
| 55 | 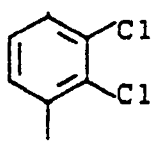 | 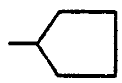 | $CH_3$ | 266 |
| 56 | 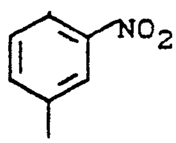 | $-CH_2-C\equiv CH$ | $C_2H_5$ | 170 |
| 57 | 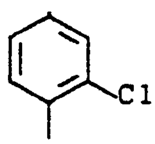 | $-CH_2-C\equiv CH$ | $CH_3$ | 202 |
| 58 | 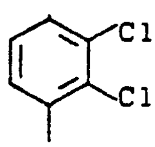 | $-CH(CH_3)_2$ | $CH_3$ | 239 |
| 59 | 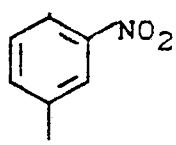 | $-CH(CH_3)_2$ | $C_2H_5$ | 146 |

Tabelle 3 (Fortsetzung)

| Bsp. Nr. | $R^3$ | $R^4$ | $R^5$ | Schmp. (°C) |
|---|---|---|---|---|
| 60 | 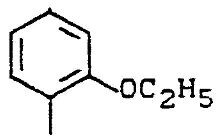 | $-CH(CH_3)_2$ | $C_2H_5$ | 209 (Z) |
| 61 | 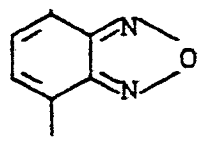 | $-CH(CH_3)_2$ | $C_2H_5$ | Harz |
| 62 | 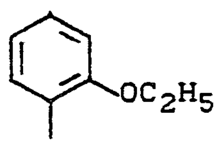 | 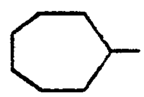 | $C_2H_5$ | 194 |
| 63 | 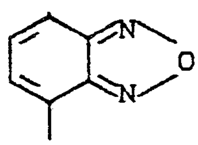 | 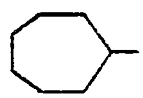 | $C_2H_5$ | Harz |
| 64 | 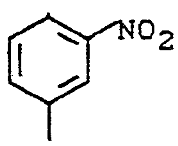 | 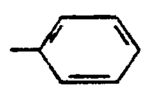 | $C_2H_5$ | 156 |
| 65 | 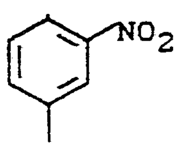 | 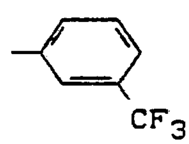 | $C_2H_5$ | 145 |
| 66 | 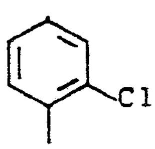 | 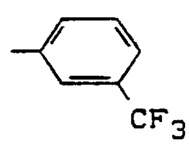 | $CH_3$ | 182 |

Tabelle 3 (Fortsetzung)

| Bsp. Nr. | $R^3$ | $R^4$ | $R^5$ | Schmp. (°C) |
|---|---|---|---|---|
| 67 | 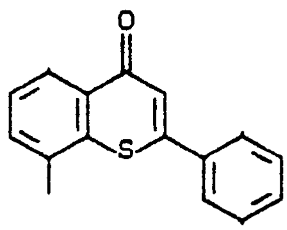 | 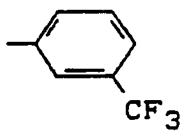 | $CH_3$ | 240 (Z) |
| 68 | 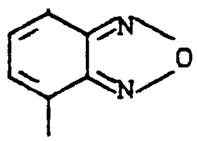 | 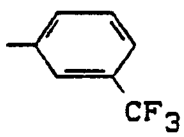 | $CH_3$ | Harz |

**Ansprüche**

1. 1,4-Dihydropyridin-3-carbonsäure-piperazide der allgemeinen Formel (I)

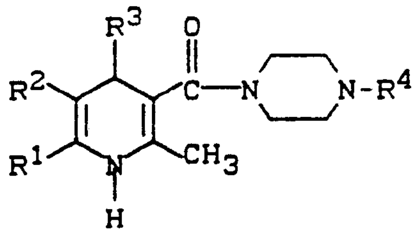

(I)

in welcher
$R^1$ -für Cyano oder
-für geradkettiges oder verzweigten $C_1$-$C_6$-Alkyl steht, das gegebenenfalls durch Halogen, Hydroxy oder $C_2$-$C_7$-Acyloxy substituiert ist,
$R^2$ -für Wasserstoff
-für Cyano,
-für Nitro oder
- für die Gruppe $CO_2R^5$ steht,
wobei
$R^5$ -geradkettiges oder verzweigtes $C_1$-$C_8$-Alkyl, bedeutet, das gegebenenfalls durch Hydroxy, Halogen, Cyano, $C_1$-$C_8$-Alkoxy, $C_1$-$C_8$-Alkylthio, Phenyl, Amino, $C_1$-$C_6$-Alkylamino, Di-$C_1$-$C_6$-alkylamino, Phenylamino, $C_2$-$C_7$-Acylamino oder $C_1$-$C_6$-Alkyl-benzyl-amino substituiert ist,
$R^3$ -für $C_6$-$C_{12}$-Aryl steht, das gegebenenfalls ein-oder mehrfach gleich oder verschieden durch Halogen, Nitro, Cyano, $C_1$-$C_8$-Alkyl, $C_1$-$C_8$-Alkoxy, $C_1$-$C_8$-Alkylthio, $C_7$-$C_{14}$-Aralkyl, $C_7$-$C_{14}$-Aralkoxy oder $C_7$-$C_{14}$-Aralkylthio, wobei die Arylreste jeweils wieder durch Nitro, Trifluormethyl, Cyano, Methoxy, Methylthio, Trifluormethoxy, Difluormethoxy, Halogen, oder $C_1$-$C_6$-Alkyl substituiert sein können, durch Trifluormethyl, Trifluormethylthio, Trifluormethoxy, Difluormethoxy, Amino, $C_1$-$C_6$-Alkylamino, Di-$C_1$-C-alkylamino oder durch $C_2$-$C_7$-Acylamino substituiert ist, oder
-für einen Heterocyclus aus der Reihe Pyridyl, Thienyl, Furyl, Pyrrolyl, Chinolyl, Isochinolyl, Pyrimidyl, Benzoxadiazolyl, Imidazolyl, Thiazolyl, Oxazolyl, Chromenyl, oder Thiochromenyl steht, wobei der Heterocyclus gegebenenfalls durch Phenyl, $C_1$-$C_6$-Alkyl, $C_1$-$C_6$-Alkoxy, $C_1$-$C_6$-Alkylthio, Trifluormethyl, Trifluormethoxy, Trifluormethylthio, Halogen, Amino, $C_1$-$C_6$-Alkylamimo, Di-$C_1$-$C_6$-alkylamino oder $C_2$-$C_7$-Acylamino substituiert ist,
und
$R^4$ -für $C_6$-$C_{12}$-Aryl steht, das gegebenenfalls ein-oder mehrfach gleich oder verschieden durch Halogen,

Nitro, Cyano, $C_1$-$C_8$-Alkyl, $C_1$-$C_8$-Alkoxy, $C_1$-$C_8$-Alkylthio, Trifluormethyl, Trifluormethoxy, Trifluormethylthio, Difluormethoxy, Hydroxy, Amino, $C_1$-$C_6$-Alkylamino, Di-$C_1$-$C_6$-alkylamino oder $C_2$-$C_7$-Acylamino substituiert ist, oder

-für geradkettiges, verzweigtes oder cyclisches $C_1$-$C_{20}$-Alkyl, $C_2$-$C_{20}$-Alkenyl oder $C_2$-$C_{20}$-Alkinyl steht, das gegebenenfalls durch Halogen, $C_1$-$C_8$-Alkoxy, $C_1$-$C_8$-Alkylthio, Amino, $C_1$-$C_8$-Alkylamino, Di-$C_1$-$C_8$-alkylamino, $C_2$-$C_7$-Acylamino, Piperidino, Piperazino, Morpholino, Thiomorpholino, Pyrrolidino oder durch $C_6$-$C_{12}$-Aryl, wobei Aryl wiederum einen oder mehrere Substituenten aus der Gruppe Nitro, Cyano, Trifluormethyl, Halogen, $C_1$-$C_8$-Alkyl, $C_1$-$C_8$-Alkylthio oder $C_1$-$C_8$-Alkoxy tragen kann, oder durch die Gruppe $COR^6$ substituiert ist, wobei

$R^6$ -für Wasserstoff,

-für Hydroxy,

-für $C_1$-$C_8$-Alkoxy oder

-für die Gruppe

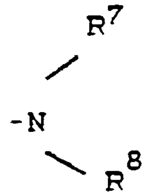

steht,

wobei

$R^7$, $R^8$ -gleich oder verschieden sind und

-Wasserstoff,

-$C_1$-$C_8$-Alkyl,

-$C_6$-$C_{12}$-Aryl,

-$C_6$-$C_{14}$-Aralkyl oder

-$C_2$-$C_7$-Acyl bedeuten oder

$R^7$ und $R^8$ gemeinsam einen 5-7 gliedrigen gesättigten oder ungesättigten Ring bilden, der als weitere Heteroatome Stickstoff, Schwefel und/oder Sauerstoff enthalten kann,

$R^4$ -für die Gruppe $COR^6$ steht, wobei $R^6$ die oben angegebene Bedeutung hat,

in Form ihrer Isomeren, Isomerengemische, Racemate, optischen Antipoden sowie deren physiologisch unbedenklichen Salze.

2. Dihydropyridin gemäß Anspruch 1, in welchem

$R^1$ -für geradkettiges oder verzweigtes $C_1$-$C_4$-Alkyl steht, das gegebenenfalls durch ein oder mehrere Fluor, Chlor, Brom, Hydroxy, Benzoyloxy oder Acetyloxy substituiert ist,

$R^2$ -für Cyano,

-für Nitro oder

-für die Gruppe $CO_2R^5$ steht,

wobei

$R^5$ -geradkettiges oder verzweigtes $C_1$-$C_6$-Alkyl bedeutet, das gegebenenfalls durch ein oder mehrere Fluor, Chlor, Brom, Cyano, Phenyl, $C_1$-$C_6$-Alkoxy, $C_1$-$C_6$-Alkylthio, Amino, $C_1$-$C_4$-Alkylamino, Di-$C_1$-$C_4$-alkylamino, Acetylamino oder Benzylmethylamino substituiert ist,

$R^3$ -für Phenyl steht, das gegebenenfalls bis zu vierfach gleich oder verschieden durch Fluor, Chlor, Brom, Nitro, Cyano, $C_1$-$C_6$-Alkyl, $C_1$-$C_6$-Alkoxy, $C_1$-$C_6$-Alkylthio, Benzyl, Benzyloxy, Benzylthio, wobei die Phenylreste jeweils wiederum durch Nitro, Trifluormethyl, Trifluormethoxy, Trifluormethylthio, Methoxy, Fluor, Chlor, Brom, oder $C_1$-$C_4$-Alkyl substituiert sein können, oder durch Trifluormethyl, Trifluormethoxy, Difluormethoxy Amino, $C_1$-$C_4$-Alkylamino, Di-$C_1$-$C_4$-alkylamino oder Acetylamino substituiert ist, oder

-für Pyridyl, Thienyl, Furyl, Pyrimidyl, Benzoxadiazolyl, 2-Phenyl-thiochromen-8-yl oder Chinolyl steht, und

$R^4$ -für Phenyl steht, das gegebenenfalls bis zu vierfach gleich oder verschiden durch Fluor, Chlor, Brom, Nitro, Cyano, $C_1$-$C_6$-Alkyl, $C_1$-$C_6$-Alkoxy, $C_1$-$C_6$-Alkylthio, Trifluormethyl, Trifluormethoxy, Difluormethoxy, Hydroxy, Amino, $C_1$-$C_3$-Alkylamino, Di-$C_1$-$C_3$-alkylamino oder Acetylamino substituiert ist, oder

-für geradkettiges, verzweigtes oder cyclisches $C_1$-$C_{15}$-Alkyl, $C_2$-$C_{15}$-Alkenyl oder $C_2$-$C_{15}$-Alkinyl steht, das gegebenenfalls durch Fluor, Chlor, Brom, $C_1$-$C_6$-Alkoxy, $C_1$-$C_6$-Alkylthio, Amino, $C_1$-$C_6$-Alkylamino, Di-$C_1$-$C_6$-Alkylamino, Acetylamino, Benzoylamino, oder durch Phenyl, wobei der Phenylrest wiederum ein bis drei Substituenten aus der Gruppe Nitro, Trifluormethyl, Fluor, Chlor, Brom, $C_1$-$C_6$-Alkyl, $C_1$-$C_6$-Alkoxy, $C_1$.$C_6$-

Alkylthio tragen kann, oder durch die Gruppe $COR^6$ substituiert ist,
wobei
$R^6$ -für Wasserstoff,
-für Hydroxy,
-für $C_1$-$C_6$-Alkoxy oder
-für die Gruppe

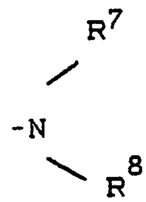

steht,
wobei
$R^7$, $R^8$ -gleich oder verschieden sind und
-für Wasserstoff,
-für $C_1$-$C_6$-Alkyl,
-für Phenyl,
-für Benzyl,
-für Acetyl oder für Benzoyl stehen
oder
$R^7$ und $R^8$ gemeinsam mit dem Stickstoffatom einen Heterocyclus aus der Reihe Pyrrolidin, Piperidin, Morpholin, Thiomorpholin, Imidazolidin, Piperazin oder Pyrrolin bilden.
oder
$R^4$ für die Gruppe der Formel $COR^6$ steht, wobei $R^6$ die oben angegebene Bedeutung hat,
in Form ihrer Isomeren, Isomerengemische, Racemate, optischen Antipoden sowie deren physiologisch unbedenklichen Salze.

3. Dihydropyridin gemäß Anspruch 1, in welchem
$R^1$ für Methyl oder Ethyl steht, das gegebenenfalls durch Chlor, Brom oder Acetyloxy substituiert ist,
$R^2$ -für Cyano,
-für Nitro oder
-für die Gruppe $CO_2R^5$ steht,
wobei
$R^5$ geradkettiges oder verzweigtes $C_1$-$C_4$-Alkyl bedeutet, das gegebenenfalls durch ein oder mehrere Fluor, Cyano, $C_1$-$C_4$-Alkoxy, $C_1$-$C_4$-Alkylthio oder N-Benzylmethylamino substituiert ist,
$R^3$ -für Phenyl steht, das gegebenenfalls bis zu dreifach gleich oder verschieden durch Fluor, Chlor, Nitro, Cyano, $C_1$-$C_4$-Alkyl, $C_1$-$C_4$-Alkoxy, $C_1$-$C_4$-Alkylthio, Benzyl, Benzyloxy, Benzylthio substituiert ist, wobei die Phenylreste wiederum bis zu zweifach durch Nitro, Trifluormethyl, Methoxy, Fluor, Chlor, Methyl oder Ethyl substituiert sein können, oder durch Trifluormethyl oder Trifluormethoxy substituiert ist, oder -für Pyridyl, Thienyl, Furyl, Pyrimidyl oder Benzoxadiazolyl oder 2-Phenyl-thiochromen-8-yl steht,
und
$R^4$ -für Phenyl steht, das gegebenenfalls bis zu zweifach gleich oder verschieden durch Fluor, Chlor, Nitro, $C_1$-$C_4$-Alkyl, $C_1$-$C_4$-Alkoxy, $C_1$-$C_4$-Alkylthio, Trifluormethoxy substituiert ist, oder
-für geradkettiges, verzweigtes oder cyclisches $C_1$-$C_{10}$-Alkyl, $C_2$-$C_{10}$-Alkenyl oder $C_2$-$C_{10}$-Alkinyl steht, das gegebenenfalls durch Fluor, Chlor, $C_1$-$C_4$-Alkoxy, $C_1$-$C_4$-Alkylthio, Amino, $C_1$-$C_4$-Alkylamino, Di-$C_1$-$C_4$-alkylamino, Acetylamino oder durch Phenyl, wobei Phenyl wiederum ein bis zwei Substituenten aus der Gruppe Nitro, Trifluormethyl, Fluor, Chlor, $C_1$-$C_4$-Alkoxy oder $C_1$-$C_4$-Alkyl tragen kann, oder durch die Gruppe $COR^6$ substituiert ist,
wobei
$R^6$ -für Wasserstoff,
-für Hydroxy,
-für $C_1$-$C_4$-Alkoxy oder
-für die Gruppe

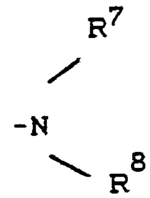

steht,
wobei
$R^7$, $R^8$ -gleich oder verschieden sind und
-für Wasserstoff,
-für $C_1$-$C_4$-Alkyl,
-für Phenyl,
-für Benzyl oder
-für Acetyl stehen, oder
$R^7$ und $R^8$ gemeinsam mit dem Stickstoffatom einen Heterocyclus aus der Reihe Pyrrolidin, Piperidin, Morpholin oder Thiomorpholin bilden, oder
$R^4$ -für die Gruppe $COR^6$ steht, wobei $R^6$ die oben angegebenen Bedeutung hat
in Form ihrer Isomeren, Isomerengemische, Racemate, optische Antipoden sowie deren physiologisch unbedenklichen Salze.

4. Dihydropyridin gemäß Ansprüche 1 bis 3 zur Bekämpfung von Erkrankungen.

5. Arzneimittel enthaltend als Wirkstoff ein Dihydropyridin gemäß Ansprüche 1 bis 3.

6. Verfahren zur Herstellung von 1,4-Dihydropyridin-3-carbonsäure-piperaziden der allgemeinen Formel (I)

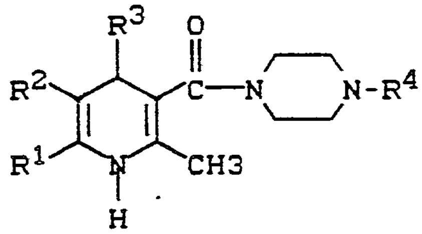

(I)

in welcher
$R^1$ -für Cyano oder
-für geradkettiges oder verzweigten $C_1$-$C_6$-Alkyl steht, das gegebenenfalls durch Halogen, Hydroxy oder $C_2$-$C_7$-Acyloxy substituiert ist,
$R^2$ -für Wasserstoff
-für Cyano,
-für Nitro oder
-für die Gruppe $CO_2R^5$ steht,
wobei
$R^5$ - geradkettiges oder verzweigtes $C_1$-$C_8$-Alkyl, bedeutet, das gegebenenfalls durch Hydroxy, Halogen, Cyano, $C_1$-$C_8$-Alkoxy, $C_1$-$C_8$-Alkoxy, $C_1$-$C_8$-Alkylthio, Phenyl, Amino, $C_1$-$C_6$-Alkylamino, Di-$C_1$-$C_6$-alkylamino, Phenylamino, $C_2$-$C_7$-Acylamino oder $C_1$-$C_6$-Alkyl-benzyl-amino substituiert ist,
$R^3$ -für $C_6$-$C_{12}$-Aryl steht, das gegebenenfalls ein-oder mehrfach gleich oder verschieden durch Halogen, Nitro, Cyano, $C_1$-$C_8$-Alkyl, $C_1$-$C_8$-Alkoxy, $C_1$-$C_8$-Alkylthio, $C_7$-$C_{14}$-Aralkyl, $C_7$-$C_{14}$-Aralkoxy oder $C_7$-$C_{14}$-Aralkylthio, wobei die Arylreste jeweils wieder durch Nitro, Trifluormethyl, Cyano, Methoxy, Methylthio, Trifluormethoxy, Difluormethoxy, Halogen, oder $C_1$-$C_6$-Alkyl substituiert sein können, durch Trifluormethyl, Trifluormethylthio, Trifluormethoxy, Difluormethoxy, Amino, $C_1$-$C_6$-Alkylamino, Di-$C_1$-$C_6$-Alkylamino oder durch $C_2$-$C_7$-Acylamino substituiert ist, oder
-für einen Heterocyclus aus der Reihe Pyridyl, Thienyl, Furyl, Pyrrolyl, Chinoly, Isochinclycl, Pyrimidyl, Benzoxadiazolyl, Imidazolyl, Thiazolyl, Oxazolyl, Chromenyl, oder Thiochromenyl steht, wobei der Heterocyclus gegebenenfalls durch Phenyl, $C_1$-$C_6$-Alkyl, $C_1$-$C_6$-Alkoxy, $C_1$-$C_6$-Alkylthio, Trifluormethyl, Trifluormethoxy, Trifluormethylthio, Halogen, Amino, $C_1$-$C_6$-Alkylamimo Di-$C_1$-$C_6$-alkylamino oder $C_2$-$C_7$-Acylamino substituiert ist,
und
$R^4$ -für $C_6$-$C_{12}$-Aryl steht, das gegebenenfalls ein-oder mehrfach gleich oder verschieden durch Halogen, Nitro, Cyano, $C_1$-$C_8$-Alkyl, $C_1$-$C_8$-Alkoxy, $C_1$-$C_8$-Alkylthio, Trifluormethyl, Trifluormethoxy, Trifluormethylthio,

Difluormethoxy, Hydroxy, Amino, $C_1$-$C_6$-Alkylamino, Di-$C_1$-$C_6$-alkylamino oder $C_2$-$C_7$-Acylamino substituiert ist, oder

-für geradkettiges, verzweigtes oder cyclisches $C_1$-$C_{20}$-Alkyl, $C_2$-$C_{20}$-Alkenyl oder $C_2$-$C_{20}$-Alkinyl steht, das gegebenenfalls durch Halogen, $C_1$-$C_8$-Alkoxy, $C_1$-$C_8$-Alkylthio, Amino, $C_1$-$C_8$-Alkylamino, Di-$C_1$-$C_8$-alkylamino, $C_2$-$C_7$-Acylamino, Piperidino, Piperazino, Morpholino, Thiomorpholino, Pyrrolidino oder durch $C_6$-$C_{12}$-Aryl, wobei Aryl wiederum einen oder merhrere Substituenten aus der Gruppe Nitro, Cyano, Trifluormethyl, Halogen, $C_1$-$C_8$-Alkyl, $C_1$-$C_8$-Alkylthio oder $C_1$-$C_8$-Alkoxy, tragen kann, oder durch die Gruppe $COR^6$ substituiert ist, wobei

$R^6$ -für Wasserstoff,

-für Hydroxy,

-für $C_1$-$C_8$-Alkoxy oder

-für die Gruppe

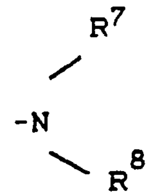

steht,

wobei

$R^7$, $R^8$ -gleich oder verschieden sind und

-Wasserstoff,

-$C_1$-$C_8$-Alkyl,

-$C_6$-$C_{12}$-Aryl,

-$C_6$-$C_{14}$-Aralkyl oder

-$C_2$-$C_7$-Acyl bedeuten oder

$R^7$ und $R^8$ gemeinsam einen 5-7 gliedrigen gesättigten oder ungesättigten Ring bilden, der als weitere Heteroatome Stickstoff, Schwefel und/oder Sauerstoff enthalten kann,

oder

$R^4$ -für die Gruppe $COR^6$ steht, wobei $R^6$ die oben angegebene Bedeutung hat,

in Form ihrer Isomeren, Isomerengemische, Racemate, optischen Antipoden sowie deren physiologisch unbedenklichen Salze, dadurch gekennzeichnet, daß man [A] Aldehyde der allgemeinen Formel (II)

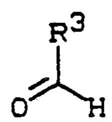

(II)

in welcher

$R^3$ die angegebene Bedeutung hat,

und Ketoverbindungen der allgemeinen Formel (III)

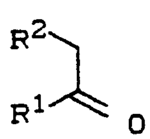

(III)

in welcher

$R^2$, $R^3$ die angegebene Bedeutung haben,

mit Ketoverbindungen der allgemeinen Formel (IV)

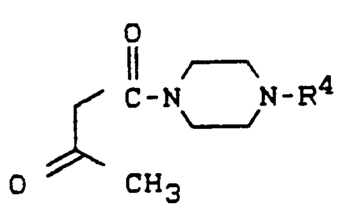

(IV)

in welcher
$R^4$ die angegebene Bedeutung hat,
und Ammoniak gegebenenfalls in Gegenwart von Wasser oder inerten organischen Lösungsmitteln umsetzt,
oder daß man
[B] Aldehyde der allgemeinen Formel (II) mit Ketoverbindungen der allgemeinen Formel (III) und Enaminen der allgemeinen Formel (V),

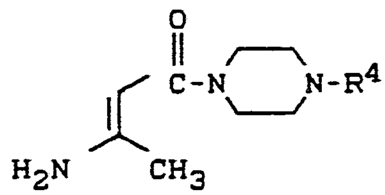

(V)

in welcher
$R^4$ die angegebene Bedeutung hat,
gegebenenfalls in Gegenwart von Wasser oder inerten organischen Lösungsmitteln umsetzt,
oder daß man
[C] Aldehyde der allgemeinen Formel (II) mit Ketoverbindungen der allgemeinen Formel (IV) und Enaminen der allgemeinen Formel (VI)

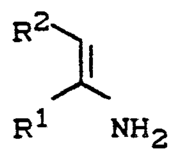

(VI)

in welcher
$R^1$, $R^2$ die angegebene Bedeutung haben,
gegebenenfalls in Gegenwart von Wasser oder interten organischen Lösungsmitteln umsetzt,
oder daß man
[D] Ketoverbindungen der allgemeinen Formel (III) und Ammoniak mit Ylidenverbindungen der allgemeinen Formel (VII)

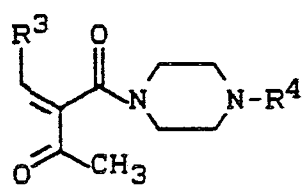

(VII)

in welcher
$R^3$, $R^4$ die oben angegebene Nedeutung haben,
gegebenenfalls in Gegenwart von Wasser oder inerten organischen Lösungsmitteln umsetzt,
oder daß man
[E] Ketoverbindungen der allgemeinen Formel (IV) und Ammoniak mit Ylidenverbindungen der allgemeinen Formel (VIII)

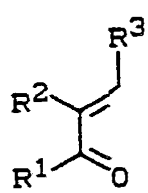

(VIII)

in welcher
$R^1$-$R^3$ die angegebene Bedeutung haben,
gegebenenfalls in Gegenwart von Wasser oder inerten organischen Lösungsmitteln umsetzt,

oder daß man

[F] Enamine der allgemeinen Formel (VI) mit Ylidenverbindungen der allgemeinen Formel (VII) gegebenenfalls in Gegenwart von Wasser oder inerten organischen Lösungsmitteln umsetzt,

oder daß man

[G] Enamine der allgemeinen Formel (V) mit Ylidenverbindungen der allgemeinen Formel (VIII) gegebenenfalls in Gegenwart von Wasser oder inerten organischen Lösungsmitteln umsetzt,

oder daß man

[H] 1.4-Dihydropyridincarbonsäuren der allgemeinen Formel (IX),

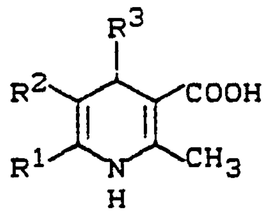

(IX),

in welcher

$R^1$-$R^3$ die angegebene Bedeutung haben,

nach bekannten Methoden, gegebenenfalls über ein reaktives Säurederivat, mit Verbindungen der allgemeinen Formel (X)

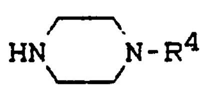

(X),

in welcher

$R^4$ die angegebene Bedeutung hat,

gegebenenfalls in Gegenwart eines inerten organischen Lösungsmittels umsetzt.

7. Verfahren nach Anspruch 6 zur Herstellung von Dihydropyridinen der Formel I, in welcher,

$R^1$ -für geradkettiges oder verzweigtes $C_1$-$C_4$-Alkyl steht, das gegebenenfalls durch ein oder mehrere Fluor, Chlor, Brom, Hydroxy, Benzoyloxy oder Acetyloxy substituiert ist,

$R^2$ -für Cyano,

-für Nitro oder

-für die Gruppe $CO_2R^5$ steht,

wobei

$R^5$ -geradkettiges oder verzweigtes $C_1$-$C_6$-Alkyl bedeutet, das gegebenenfalls durch ein oder mehrere Fluor, Chlor, Brom, Cyano, Phenyl, $C_1$-$C_6$-Alkoxy, $C_1$-$C_6$-Alkylthio, Amino, $C_1$-$C_4$-Alkylamino, Di-$C_1$-$C_4$-alkylamino, Acetylamino oder Benzylmethylamino substituiert ist,

$R^3$ -für Phenyl steht, das gegebenenfalls bis zu vierfach gleich oder verschieden durch Fluor, Chlor, Brom, Nitro, Cyano, $C_1$-$C_6$-Alkyl, $C_1$-$C_6$-Alkoxy, $C_1$-$C_6$-Alkylthio, Benzyl, Benzyloxy, Benzylthio, wobei die Phenylreste jeweils wiederum durch Nitro, Trifluormethyl, Trifluormethoxy, Trifluormethylthio, Methoxy, Fluor, Chlor, Brom, oder $C_1$-$C_4$-Alkyl substituiert sein können, oder durch Trifluormethyl, Trifluormethoxy, Difluormethoxy, Amino, $C_1$-$C_4$-Alkylamino, Di-$C_1$-$C_4$-alkylamino oder Acetylamino substituiert ist, oder

-für Pyridyl, Thienyl, Furyl, Pyrimidyl, Benzoxadiazolyl, 2-Phenyl-thiochromen-8-yl oder Chinolyl steht,

und

$R^4$ -für Phenyl steht, das gegebenenfalls bis zu vierfach gleich oder verschiden durch Fluor, Chlor, Brom, Nitro, Cyano, $C_1$-$C_6$-Alkyl, $C_1$-$C_6$-Alkoxy, $C_1$-$C_6$-Alkylthio, Trifluormethyl, Trifluormethoxy, Difluormethoxy, Hydroxy, Amino, $C_1$-$C_3$-Alkylamino, Di-$C_1$-$C_3$-alkylamino oder Acetylamino substituiert ist, oder

-für geradkettiges, verzweigtes oder cyclisches $C_1$-$C_{15}$-Alkyl, $C_2$-$C_{15}$-Alkenyl oder $C_2$-$C_{15}$-Alkinyl steht, das gegebenenfalls durch Fluor, Chlor, Brom, $C_1$-$C_6$-Alkoxy, $C_1$-$C_6$-Alkylthio, Amino, $C_1$-$C_6$-Alkylamino, Di-$C_1$-$C_6$-Alkylamino, Acetylamino, Benzoylamino, oder durch Phenyl, wobei der Phenylrest wiederum ein bis drei Substituenten aus der Gruppe Nitro, Trifluormethyl, Fluor, Chlor, Brom, $C_1$-$C_6$-Alkyl, $C_1$-$C_6$-Alkoxy, $C_1$-$C_6$-Alkylthio tragen kann, oder durch die Gruppe $COR^6$ substituiert ist,

wobei

$R^6$ -für Wasserstoff,

-für Hydroxy,
-für $C_1$-$C_6$-Alkoxy oder
-für die Gruppe

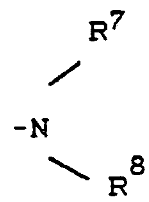

steht,
wobei
$R^7$, $R^8$ -gleich oder verschieden sind und
-für Wasserstoff,
-für $C_1$-$C_6$-Alkyl,
-für Phenyl,
-für Benzyl,
-für Acetyl oder für Benzoyl stehen
oder
$R^7$ und $R^8$ gemeinsam mit dem Stickstoffatom einen Heterocyclus aus der Reihe Pyrrolidin, Piperidin, Morpholin, Thiomorpholin, Imidazolidin, Piperazin oder Pyrrolin bilden.
oder
$R^4$ für die Gruppe der Formel $COR^6$ steht, wobei $R^6$ die oben angegebene Bedeutung hat,
in Form ihrer Isomeren, Isomerengemische, Racemate, optitischen Antipoden sowie deren physiologisch unbedenklichen Salze.

8. Verfahren nach Anspruch 6 zur Herstellung von Dihydropyridinen der Formel (I), in welcher
$R^1$ für Methyl oder Ethyl steht, das gegebenenfalls durch Chlor, Brom oder Acetyloxy substituiert ist,
$R^2$ -für Cyano,
-für Nitro oder
-für die Gruppe $CO_2R^5$ steht,
wobei
$R^5$ geradkettiges oder verzweigtes $C_1$-$C_4$-Alkyl bedeutet, das gegebenenfalls durch ein oder mehrere Fluor, Cyano, $C_1$-$C_4$-Alkoxy, $C_1$-$C_4$-Alkylthio oder N-Benzylmethylamino substituiert ist,
$R^3$ -für Phenyl steht, das gegebenenfalls bis zu dreifach gleich oder verschieden durch Fluor, Chlor, Nitro, Cyano, $C_1$-$C_4$-Alkyl, $C_1$-$C_4$-Alkoxy, $C_1$-$C_4$-Alkylthio, Benzyl, Benzyloxy, Benzylthio substituiert ist, wobei die Phenylreste wiederum bis zu zweifach durch Nitro, Trifluormethyl, Methoxy, Fluor, Chlor, Methyl oder Ethyl substituiert sein können, oder durch Trifluormethyl oder Trifluormethoxy substituiert ist, oder -für Pyridyl, Thienyl, Furyl, Pyrimidyl oder Benzoxadiazolyl oder 2-Phenyl-thiochromen-8-yl steht,
und
$R^4$ -für Phenyl steht, das gegebenenfalls bis zu zweifach gleich oder verschieden durch Fluor, Chlor, Nitro, $C_1$-$C_4$-Alkyl, $C_1$-$C_4$-Alkoxy, $C_1$-$C_4$-Alkylthio, Trifluormethoxy substituiert ist, oder
-für geradkettiges, verzweigtes oder cyclisches $C_1$-$C_{10}$-Alkyl, $C_2$-$C_{10}$-Alkenyl oder $C_2$-$C_{10}$-Alkinyl steht, das gegebenenfalls durch Fluor, Chlor, $C_1$-$C_4$-Alkoxy, $C_1$-$C_4$-Alkylthio, Amino, $C_1$-$C_4$-Alkylamino, Di-$C_1$-$C_4$-alkylamino, Acetylamino oder durch Phenyl, wobei Phenyl wiederum ein bis zwei Substituenten aus der Gruppe Nitro, Trifluormethyl, Fluor, Chlor, $C_1$-$C_4$-Alkoxy oder $C_1$-$C_4$-Alkyl tragen kann, oder durch die Gruppe $COR^6$ substituiert ist,
wobei
$R^6$ -für Wasserstoff,
-für Hydroxy,
-für $C_1$-$C_4$-Alkoxy oder
-für die Gruppe

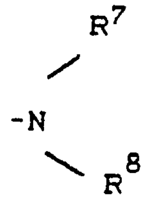

steht,
wobei
$R^7$, $R^8$ -gleich oder verschieden sind und
-für Wasserstoff,
-für $C_1$-$C_4$-Alkyl,
-für Phenyl,
-für Benzyl oder
-für Acetyl stehen, oder
$R^7$ und $R^8$ gemeinsam mit dem Stickstoffatom einen Heterocyclus aus der Reihe Pyrrolidin, Piperidin, Morpholin oder Thiomorpholin bilden, oder
$R^4$ -für die Gruppe $COR^6$ steht, wobei $R^6$ die oben angegebenen Bedeutung hat
in Form ihrer Isomeren, Isomerengemische, Racemate, optische Antipoden sowie deren physiologisch unbedenklichen Salze.

9. Verfahren zur Herstellung von Arzneimitteln enthaltend ein Dihydropyridin der allgemeinen Formel I in Anspruch 6, dadurch gekennzeichnet, daß man den Wirkstoff mit üblichen Hilfs-und Trägerstoffen in bekannter Weise mischt.

10. Verwendung der Dihydropyridine gemäß Ansprüchen 1-3 zur Herstellung von Arzneimitteln mit Koronarwirkung, zur Beeinflussung des Blutdruckes und zur Normalisierung des Blutzuckers.

Europäisches Patentamt

# EUROPÄISCHER RECHERCHENBERICHT

Nummer der Anmeldung

EP 87 10 0174

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-3 445 356 (CIBA-GEIGY AG)<br>* Ansprüche 1-3, 24-30, Seite 16, Zeilen 1-6 * | 1,4-6, 9,10 | C 07 D 211/90<br>C 07 D 413/04<br>C 07 D 409/04<br>C 07 D 401/04<br>C 07 D 405/04<br>C 07 D 417/04<br>A 61 K 31/445//<br>C 07 D 295/18 |
| A | EP-A-0 083 315 (CIBA-GEIGY AG)<br>* Ansprüche 1-3, 45-51, Seite 7, Zeilen 19-24 * | 1,4-6, 9,10 | |
| A,D | DE-A-2 228 377 (BAYER AG)<br>* Ansprüche * | 1,4-6, 9,10 | |
| A | EP-A-0 111 453 (CIBA-GEIGY AG)<br>* Ansprüche 1-3, 21-27, Seite 11, Zeilen 21-26 * | 1,4-6, 9,10 | **RECHERCHIERTE SACHGEBIETE (Int. Cl.4)** |
| A | EP-A-0 123 112 (BAYER AG)<br>* Ansprüche 1, 4-10 * | 1,4-6, 9,10 | C 07 D 211/00<br>C 07 D 413/00<br>C 07 D 409/00<br>C 07 D 401/00<br>C 07 D 405/00<br>C 07 D 417/00<br>A 61 K 31/00 |
| A | EP-A-0 097 821 (PIERREL S.P.A.)<br>* Ansprüche 1, 20, 21 * | 1,4-6, 9,10 | |

-----

Der vorliegende Recherchenbericht wurde für alle Patentansprüche erstellt.

| Recherchenort | Abschlußdatum der Recherche | Prüfer |
|---|---|---|
| BERLIN | 02-04-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument